# EUROPEAN PATENT APPLICATION

(11) **EP 4 467 573 A1**
(43) Date of publication of application: **27.11.2024**
(21) Application number: 22880438.1
(22) Date of filing: 15.10.2022
(51) Int. Cl.: C07K 19/00, C07K 16/18, A61K 39/395

(54) **BISPECIFIC BINDING MOLECULE**

(30) Priority: 15.10.2021 CN 202111204907
(71) Applicant: Yichen Therapeutics Limited, Wan Chaihk, Hong Kong (HK)
(72) Inventor: WANG, Feng, Suzhou, Jiangsu 215000 (CN); CHEN, Na, Suzhou, Jiangsu 215000 (CN)
(74) Representative: Dehns Germany Partnerschaft mbB
(86) International application number: PCT/CN2022/125540
(87) International publication number: WO 2023/061502

(57) **Abstract**

A bispecific binding molecule having high selectivity for Kv1.3 but different affinity for integrin β7, a pharmaceutical composition thereof, and a use thereof. The bispecific binding molecule is formed by the fusion of a toxin polypeptide that binds to a Kv1.3-type potassium ion channel and heavy chain or light chain variable region CDRs of an antibody that specifically binds to integrin β7. The obtained bispecific binding molecule has higher selectivity for Kv1.3^{high} β7^{high} T cells (relative to Kv1.3^{low} β7^{high} T cells), and can greatly improve the therapeutic efficacy, while preventing possible adverse reactions caused by other cells that positively express integrin β7. The described difference in binding activity toward two targets enables the bispecific binding molecule to have a binding preference for the two targets, thereby having certain advantages in the design of antibody drugs.

## Description

### Technical field

This invention relates to a bispecific binding molecule, specifically a bispecific binding molecule with high selectivity for Kv1.3 and differing affinities for integrin β7.

### Background

Over the past 20 years, with the development of genetic engineering technologies, over 50 different forms of bispecific binding molecules have been designed, such as IgG fusion, quadromas, diabodies, tandem single-chain variable fragments (ScFvs), DART, knobs-into-holes, DVD-Ig, etc. (Nat Rev Drug Discov 2019, 18 (8), 585-608; Drug Discov Today 2015, 20 (7), 838-847). Generally, bispecific binding molecules can be divided into two categories: IgG-based bispecific binding molecules, and non-IgG antibody types (Drug Discovery Today 2015, 20 (7), 838-847). Regardless of the type, It is necessary to link two antibody's variable region fragments with binding and functional specificity by chemical or genetic engineering method to form a bispecific binding molecule in a framework . Currently, more than 30 bispecific binding molecules have entered clinical trials, but increasing evidence suggests that each framework form has its limitations (Drug Des Devel Ther 2018, 12, 195-208).

Inflammatory bowel disease (IBD) is a chronic inflammatory disease associated with abnormal immune responses to intestinal bacteria. Alleviating intestinal tissue inflammation, restoring intestinal barrier function, and improving the balance of the gastrointestinal immune system are crucial for the cure of the disease (Inflamm Bowel Dis 2020, 26, 1131-1143). Although the pathogenesis of IBD is not fully understood, autoimmunity is believed to play a key role in it. Autoreactive memory T cells are considered a major cause of many autoimmune diseases, including multiple sclerosis (MS), type 1 diabetes (T1D), IBD, rheumatoid arthritis (RA), psoriasis, and systemic lupus erythematosus (SLE). Targeting autoreactive effector memory T (T_{EM}) cells has been considered to be a promising strategy for many immunomodulatory therapies for such diseases (Immunol Res 2013, 57, 12-22). The voltage-gated potassium channel Kv1.3 and the calcium-activated K+ channel KCa3.1 are essential for T cell membrane potential and calcium signaling and have been identified as potential therapeutic targets for immunosuppression. When Kv1.3 is activated, its expression is selectively upregulated on autoreactive effector memory T cells. Therefore, inhibiting Kv1.3 can selectively target pathogenic T_{EM} cells in all T cell-mediated autoimmune diseases while avoiding immune responses associated with naive and central memory T (T_{CM}) cells. Some animal toxins are natural inhibitors of the Kv1.3 channel with high selectivity and efficacy, showing potential to be developed into drugs to inhibit the activity of T_{EM} cells. For example, ShK is a toxin peptide from sea anemones that can specifically inhibit the Kv1.3 ion channel, and extensive research on ShK is currently ongoing (JBC 1998, 273, 32697-32707; Frontiers in neuroscience 2019, 13, 1393; PLOS ONE 2013, 8, e78712; Pharmacology 2009, 75, 762-773; PNAS 2013, 110, E2239). ShK-186, a synthetic analogue of ShK, has completed Phase I clinical trials for treatment of plaque psoriasis (PLOS ONE 2017, 12(7), e0180762). Another natural toxin, Vm24, extracted from Mexico scorpions, is also a highly selective and potent inhibitor of the Kv1.3 ion channel (Biochemistry 2012, 51, 4049-4061; Mol Pharmacol 2012, 82, 372-382). Experiments showed that Vm24 can attenuate the response of CD4+ T_{EM} cells to TCR stimulation (Cell Commun Signal: CCS 2018, 16, 45). However, these active peptides face many challenges when used directly as drugs, such as synthesis difficulties and short plasma half-lives (C. Beeton et al, PNAS 2006, 103, 17414; J Pharmacol Exp Ther 2012, 342, 642-653). Recently, the structure of the human Kv1.3 channel protein and its mutant H451N were reported, which will aid in the design and construction of Kv1.3 channel inhibitors (Cell Discov 2021, 7, 39).

Integrin β7 is an adhesion factor that is important to mediate the migration and homing of lymphocytes to gut-associated lymphoid tissue by interacting with mucosal addressin cell adhesion molecule-1 (MAdCAM-1). In the state of intestinal inflammation, the expression of MAdCAM-1 will increase, leading to more lymphocytes migrating to and residing in the inflamed tissues, exacerbating the inflammatory state of the lesion tissues and facillitating the disease progression. Etrolizumab is a monoclonal antibody against β7, which is currently undergoing clinical trials as a therapeutic drug for inflammatory bowel disease (IBD). It was reported that the adverse reactions shown in the phase II clinical trial of etrolizumab include rash, influenza-like illness, and arthralgia (Brit J Dermatol 2021, 184 (2), 270-280). Results from phase III clinical showed that etrolizumab met its primary endpoint of inducing remission versus placebo for people with ulcerative colitis in only two of three studies; Etrolizumab failed to meet its primary endpoint versus placebo as maintenance therapy in people with ulcerative colitis (https://www.roche.com/media/releases/med-cor-2020-08-10.htm). These adverse reactions of anti-β7 antibody and its insufficient efficacy also herald the need to develop more selective drugs to effectively target T_{EM} cells in IBD.

Therefore, there is a demand for bispecific binding molecules with stronger selectivity for Kv1.3 and tunable affinity for integrin β7, which can effectively reduce adverse reactions while ensuring efficacy.

### Summary

Due to the significantly higher expression of integrin β7 on naive T cells compared to that on T_{EM} cells, antibodies specifically targeting integrin β7, such as etrolizumab, exhibit a more pronounced blocking effect on naive T cells than on pathogenic T_{EM} cells. This may be the underlying reason for the limited efficacy of etrolizumab and its various adverse reactions. Our early experiment also showed that Kv1.3 inhibitors can effectively suppress the function of T_{EM} cells and alleviate inflammatory response in the delayed-type hypersensitivity (DTH) model in rats. Therefore, it is extremely important to develop a bispecific binding molecule that targets both integrin β7 and Kv1.3, but with a higher affinity for Kv1.3 rather than for β7 to avoid or limit the side effects caused by other integrin β7 highly expressed cells.

In the present invention, by grafting Vm24, a Kv1.3-inhibiting toxin peptide, into the CDRs or FRs of the heavy chain and light chain variable regions of the integrin β7 antibody, we obtained bispecific binding molecules with higher affinity for K v 1.3 and differential affinity for integrin β7. By virtue of its high affinity and selectivity for Kv1.3, the bispecific binding molecules of the present invention exhibits higher selectivity for Kv1.3 ^{high} β7 ^{high} T cells (relative to Kv1.3 ^{low} β7 ^{high} T cells), not only significantly enhancing therapeutic effect but also avoiding the possible adverse reactions caused by other integrin β7-positive cells. For this reason, the present invention provides following technical solutions:
One aspect of the present invention provides a bispecific binding molecule comprising: A) a toxin peptide that specifically binds to Kv1.3 type potassium ion channel; B) an antibody that specifically binds to integrin β7.

In some embodiments, the toxin peptide specifically binding to the Kv1.3 potassium channel is grafted into the CDRs (HCDR1, HCDR2, or HCDR3) of the heavy chain variable region of the integrin β7 antibody through a linker peptide. In one embodiment, the toxin peptide specifically binding to the K v 1.3 potassium channel is grafted into the CDRs (LCDR1, LCDR2, or LCDR3) of the light chain variable region of the integrin β7 antibody through a linker peptide.

In one embodiment, the toxin peptide specifically binding to the Kv1.3 potassium channel is grafted into the FRs (FR1H, FR2H, FR3H, or FR4H) of the heavy chain variable region of the integrin β7 antibody.

In one embodiment, the toxin peptide specifically binding to the Kv1.3 potassium channel is grafted into the FRs (FR1L, FR2L, FR3L, or FR4L) of the light chain variable region of the integrin β7 antibody.

In one embodiment, the toxin peptide specifically binding to the Kv1.3 potassium channel is Vm24. In one embodiment, the toxin peptide Vm24 has the amino acid sequence as shown in SEQ ID NO: 9. In one embodiment, the toxin peptide has an amino acid sequence with 95% homology to SEQ ID NO: 9.

In one embodiment, the antibody specifically binding to integrin β7 has LCDR1, LCDR2, LCDR3 sequences as shown in SEQ ID NO: 10, SEQ ID NO: 11, and SEQ ID NO: 12, respectively, and HCDR1, HCDR2, HCDR3 sequences as shown in SEQ ID NO: 13, SEQ ID NO: 14, and SEQ ID NO: 15, respectively.

In some embodiments, the bispecific binding molecule comprises: a) a toxin peptide specifically binds to the Kv1.3 potassium channel with the amino acid sequence as shown in SEQ ID NO: 9; b) an antibody specifically binding to integrin β7 with LCDR1, LCDR2, LCDR3, HCDR1, HCDR2, HCDR3 as shown in SEQ ID NO: 10, SEQ ID NO: 11, SEQ ID NO: 12, SEQ ID NO: 13, SEQ ID NO: 14, and SEQ ID NO: 15, respectively.

In some embodiments, the toxin peptide as shown in SEQ ID NO: 9 is grafted into the LCDR1, LCDR2, LCDR3, HCDR1, HCDR2, HCDR3, FR1L, FR2L, FR3L, FR4L, FR1H, FR2H, FR3H, or FR4H of the integrin β7 antibody.

In some embodiments, the linker peptide has the amino acid sequences as shown in GGSGAKLAALKAKLAALKGGGGS (SEQ ID NO: 16) and/or GGGGSELAALEAELAALEAGGSG (SEQ ID NO: 17).

In one embodiment, the toxin peptide as shown in SEQ ID NO: 9 is grafted into the LCDR1 of the integrin β7 antibody. In a specific embodiment, the toxin peptide as shown in SEQ ID NO: 9 is grafted between S28 (position 28, Kabat numbering, hereafter all positions are Kabat numbering) and L32 of the LCDR1 of the integrin β7 antibody. In a specific embodiment, the toxin peptide as shown in SEQ ID NO: 9 is grafted between S28 and L32 of the LCDR1 of the integrin β7 antibody using the linker peptides as shown in SEQ ID NO: 16 and SEQ ID NO: 17. In one embodiment, the bispecific binding molecule has the HC as shown in SEQ ID NO: 1 and the LC as shown in SEQ ID NO: 3. In such embodiment, the bispecific binding molecule ATF-L1 has the following characteristics: its affinity for Kv1.3 is substantially similar to that of Syn-Vm24-LCDR3; its affinity for integrin β7 is 167.1 pM, approximately 10 times weaker than that of the integrin β7 antibody (17.57 pM); its blocking effect on interaction between β7^{high} T cells and MAdCAM-1 (IC50 = 1.09 nM) is 3 times weaker than that of the integrin β7 antibody (0.42 nM); its inhibitory effect on Kv1.3 current is similar as that of the integrin β7 antibody. Furthermore, in binding selectivity experiments, the percentage of antibody bound to Kv1.3^{high}β7^{hgh} T cells is 2.4 ± 0.1, 1.7 ± 0.2, 1.3 ± 0.1, and 1.2 ± 0.1 times of antibody bound to Kv1.3^{low}β7^{high} T cells when the bispecific binding molecule is at the concentrations of 50 pM, 100 pM, 200 pM, or 400 pM, respectively; In competition experiments, Kv1.3^{low}β7^{high} T cells-bound ATF-L1-PE is more easily competed by etrolizumab (compared to Kv1.3^{high}β7^{high} T cells), indicating that the bispecific binding molecule ATF-L1 has a higher selectivity for Kv1.3 than for β7, making it more likely to bind to T cells with high Kv 1.3 expression; In the DSS mouse model, the bispecific binding molecule ATF-L1 can specifically inhibit the migration of human T cells with high K v 1.3 expression to inflamed intestinal tissues.

In one embodiment, the toxin peptide as shown in SEQ ID NO: 9 is grafted into the LCDR3 of the integrin β7 antibody. In a specific embodiment, the toxin peptide as shown in SEQ ID NO: 9 is grafted between 91G and 96N of the LCDR3 of the integrin β7 antibody. In a specific embodiment, the toxin peptide as shown in SEQ ID NO: 9 is grafted between 91G and 96N of the LCDR3 of the integrin β7 antibody using the linker peptides as shown in SEQ ID NO: 16 and SEQ ID NO: 17. In one embodiment, the bispecific binding molecule has the HC as shown in SEQ ID NO: 1 and the LC as shown in SEQ ID NO: 5. In such embodiment, the affinity of the bispecific binding molecule ATF-L3 to Kv1.3 is substantially similar to that of Syn-Vm24-LCDR3, and its affinity to integrin β7 (40.82 pM) is 2.3 times weaker than that of the integrin β7 antibody( 17.57 pM).

In one embodiment, the toxin peptide as shown in SEQ ID NO: 9 is grafted into the HCDR1 of the integrin β7 antibody. In a specific embodiment, the toxin peptide as shown in SEQ ID NO: 9 is grafted between 26 F and 32N of the HCDR1 of the integrin β7 antibody. In a specific embodiment, the toxin peptide as shown in SEQ ID NO: 9 is grafted between 26F and 32N of the HCDR1 of the integrin β7 antibody using the linker peptides as shown in SEQ ID NO: 16 and SEQ ID NO: 17. In one embodiment, the bispecific binding molecule has the HC as shown in SEQ ID NO: 6 and the LC as shown in SEQ ID NO: 2. In such embodiment, the affinity of the bispecific binding molecule ATF-H1 for Kv1.3 is substantially similar to that of Syn-Vm24-LCDR3, and its inhibitory effect on Kv1.3 potassium current (0.72 nM) is also similar to that of Syn-Vm24-LCDR3 (0.49 nM).

In one embodiment, the toxin peptide as shown in SEQ ID NO: 9 is grafted into the HCDR3 of the integrin β7 antibody. In a specific embodiment, the toxin peptide as shown in SEQ ID NO: 9 is grafted between 97R and 102G of the HCDR3 of the integrin β7 antibody. In a specific embodiment, the toxin peptide as shown in SEQ ID NO: 9 is grafted between and 102G of the HCDR3 of the integrin β7 antibody using the linker peptides as shown in SEQ ID NO: 16 and SEQ ID NO: 17. In one embodiment, the bispecific binding molecule has the HC as shown in SEQ ID NO: 8 and the LC as shown in SEQ ID NO: 2. In such embodiment, the affinity of the bispecific binding molecule ATF-H3 for Kv1.3 is substantially similar to that of Syn-Vm24-LCDR3, and its affinity for integrin β7 (13.8 8nM) is similar to that of the integrin β7 antibody (17.57 pM).

In one embodiment, the toxin peptide as shown in SEQ ID NO: 9 is grafted into the FR3L of the integrin β7 antibody. In a specific embodiment, the toxin peptide as shown in SEQ ID NO: 9 is grafted between 56S and 60S of the FR3L of the integrin β7 antibody. In a specific embodiment, the toxin peptide as shown in SEQ ID NO: 9 is grafted between 56S and 60S of the FR3L of the integrin β7 antibody using the linker peptides as shown in SEQ ID NO: 16 and SEQ ID NO: 17. In one embodiment, the bispecific binding molecule has the HC as shown in SEQ ID NO: 1 and the LC as shown in SEQ ID NO: 4. In such embodiment, the affinity of bispecific binding molecule ATF-L2 for Kv1.3 is similar to that of Syn-Vm24-LCDR3, and its affinity (35.9 pM) for integrin β7 is approximately 2 times weaker than that of the integrin β7 antibody (17.57 pM).

In one embodiment, the toxin peptide as shown in SEQ ID NO: 9 is grafted into the FR3H of the integrin β7 antibody. In a specific embodiment, the toxin peptide as shown in SEQ ID NO: 9 is grafted between 60N and 65S of the FR3H of the integrin β7 antibody. In a specific embodiment, the toxin peptide as shown in SEQ ID NO: 9 is grafted between 60N and 65S of the FR3H of the integrin β7 antibody using the linker peptides as shown in SEQ ID NO: 16 and SEQ ID NO: 17. In one embodiment, the bispecific binding molecule has the HC as shown in SEQ ID NO: 7 and the LC as shown in SEQ ID NO: 2. In such embodiments, the affinity of bispecific binding molecule ATF-H2 for Kv1.3 is similar to that of Syn-Vm24-LCDR3, and its affinity (19.25 pM) for integrin β7 is also similar to that of the integrin β7 antibody (17.57 pM); furthermore, its inhibitory effect on Kv1.3 current (0.53 pM) is similar to that of Syn-Vm24-LCDR3 (0.49 pM), and its inhibitory effect on the adhesion of β7^{high} T cells to MAdCAM-1 (0.45 pM) is also similar to that of the integrin β7 antibody (0.42 pM).

Another aspect of the present invention provides a polynucleotide encoding the aforementioned bispecific binding molecules.

Another aspect of the present invention provides a pharmaceutical composition comprising the aforementioned bispecific binding molecules and a pharmaceutically acceptable carrier.

Another aspect of the present invention provides the use of the aforementioned bispecific binding molecule in the preparation of drugs for the treatment or prevention of diseases related to the Kv1.3 potassium channel and/or integrin β7. In some embodiments, diseases related to the Kv1.3 potassium channel and/or integrin β7 include: inflammatory diseases, immune and proliferative diseases, rheumatoid arthritis (RA), ankylosing spondylitis, psoriatic arthritis, osteoarthritis, osteoporosis, uveitis, inflammatory fibrosis, scleroderma, pulmonary fibrosis, cirrhosis, inflammatory bowel disease, Crohn's disease, ulcerative colitis, asthma, allergic asthma, allergies, chronic obstructive pulmonary disease (COPD), multiple sclerosis, psoriasis, contact dermatitis, systemic lupus erythematosus (SLE) and other forms of lupus, diabetes, type I diabetes, obesity, cancer, lupus, restenosis, systemic sclerosis, scleroderma, glomerulonephritis, Sjögren's syndrome, inflammatory bone resorption, transplant rejection or graft-versus-host disease, etc.

### Brief Description of the Drawings

Figure 1 shows the SDS-PAGE of the antibody fusion proteins of Vm24 and etrolizumab.
Figure 2A presents the ELISA results of different bispecific binding molecules binding to integrin β7.Figure 3 displays the flow cytometry results of high levels of β7 on human T cells induced by PMA and ionomycin (IM).
Figure 4 illustrates the inhibitory effect of bispecific binding molecules on the adhesion between integrin β7 T cells and MAdCAM-1 .
Figure 5 demonstrates the inhibitory effect of bispecific binding molecules on Kv1.3 potassium currents by the Flipr real-time fluorescence detection method.
Figure 6 shows the flow cytometry results of Jurkat cells infected by lentivirus with Kv1.3 and EGFP expression gene cassettes, wherein A) depicts a schematic diagram of the lentiviral plasmid construction; B) shows the negative control group (the rate of Kv1.3-positive cells is close to 0) without anti-Kv1.3 antibody; C) indicates the rate of Kv1.3-positive cell when the EGFP-positivity rate is 96.74%; D) shows the rate of Kv1.3-positive cell when the EGFP positivity rate is 84.57%; E) displays the rate of Kv1.3-positive cell when the EGFP positivity rate is 20.52%.

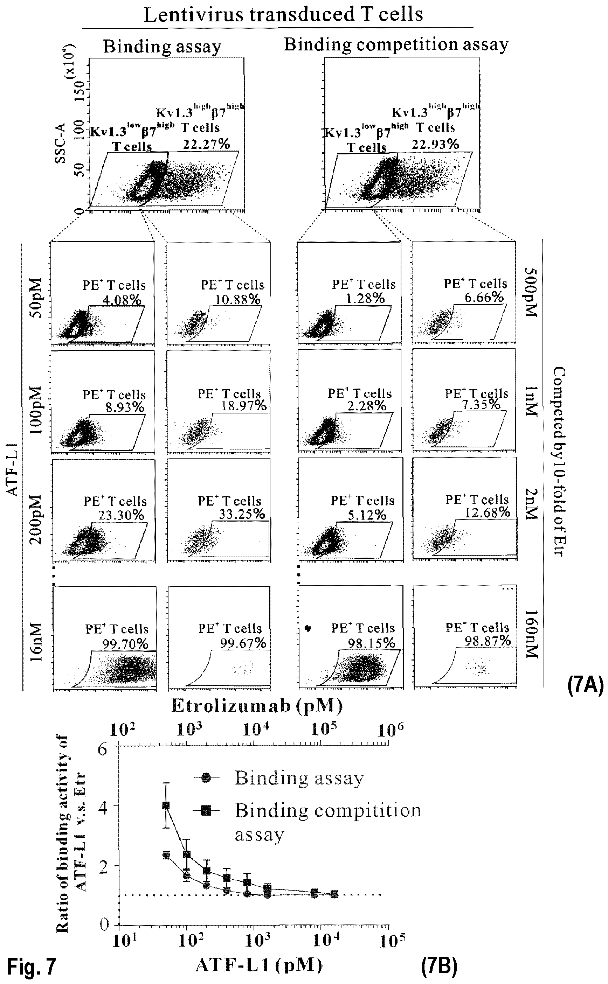

Figure 7 illustrates the binding selectivity of bispecific binding molecules on different subsets of human T cells. A presents the flow cytometry results where the left two columns show the migration rates (PE fluorescence positivity rate) of ATF-L1-PE binding to Kv1.3^{high}β7^{high} T cells (EGFP-positive cells) or Kv1.3^{low}β7^{high} T cells (EGFP-negative cells) at different concentrations, and the right two columns show the migration rates (PE fluorescence positivity rate) of ATF-L1-PE binding to Kv1.3^{high}β7^{high} T cells or Kv1.3^{low}β7^{high} T cells when monoclonal antibody etrolizumab with the concentration 10 times that of ATF-L1-PE is added; B quantitatively analyzes the ratio of the migration rates of ATF-L1 binding to Kv1.3^{high}β7^{high} T cells versus binding to Kv1.3^{low}β7^{high} T cells in binding experiments and competitive binding experiments.
Figure 8 presents in vivo activity of bispecific binding molecules; A depicts the proportion of adoptively transferred human T cells detected in the blood of DSS model animals and normal control animals using flow cytometric analysis; B displays the quantitative analysis of the adoptively transferred human T cells detected in the blood of each group with flow cytometry; C provides the statistical analysis of the two-photon imaging results of the colonic tissues.
Figure 9 displays the HE staining of the colonic tissue slices, where A shows image of colonic tissue from the normal control group treated with distilled drinking water , demonstrating intact villous structures, goblet cells (*), and crypt structures (#); B displays HE staining of the DSS group, showing the disrupted villous tissue structure, significant loss of goblet cells, and crypt structure loss; C presents the statistical analysis of Kv1.3 over-expressed T cells.
Figure 10 shows the body weight changes of mice from different treatment groups of the DSS-induced colitis model. The two doses of ATF-L1 (low-dose group and high-dose group) and the high-dose group of etrolizumab significantly alleviated the weight loss induced by DSS in mice. Significant analysis of body weight between groups on the 9th day : ATF-L1 low-dose group Vs DSS group (P<0.01, **); ATF-L1 high-dose group Vs DSS group (P<0.001, ***); Etro high-dose group Vs DSS group (P<0.01, **); DPBS blank control group VS DSS group (P<0.001, ***), where "etro" refers to etrolizumab.
Figure 11 presents the Disease Activity Index (DAI) scores of mice in each group of the DSS-induced colitis model.
Figure 12 is the comparison of colon lengths of mice in each group of the DSS-induced colitis model.

### Detailed Description of the Embodiments

This invention is herein described in detail by reference to the following definitions and embodiments. Contents of patents and publications mentioned herein, including all sequences disclosed in these patents and publications, are explicitly incorporated herein by reference.

The term "polypeptide" refers to a molecule comprising at least two amino acid residues connected by peptide bonds to form a peptide. Polypeptides can also be referred to as "proteins".

As used herein, the term "inhibitory toxin polypeptide targeting Kv1.3 potassium ion channel" refers to a polypeptide that can specifically bind to the Kv1.3 potassium ion channel and inhibit its activity. In the present invention, the " inhibitory polypeptide targeting Kv1.3 potassium ion channel" preferably refers to a novel polypeptide Vm24 isolated from the venom of the Mexican scorpion V. mexicanus. In a preferred embodiment, Vm24 has the amino acid sequence shown in SEQ ID NO: 9. Amino acid sequences having at least 90%, 95%, 96%, 97%, 98%, and 99% identity with SEQ ID NO: 9, and being capable of inhibiting K v 1.3 type potassium ion channels are also included in the present invention.

The "bispecific binding molecule" of the present invention can specifically bind to both the Kv1.3 potassium ion channel and integrin β7.

Grafting exogenous peptides into the CDRs region of an antibody often disrupts the binding of the antibody to its antigen, leading to a significant decrease or even loss of affinity between the antibody and its antigen. Typically, not all of the six CDRs of antibodies directly interact with antigens. We reason that if Vm24 is grafted into the CDRs that do not interact with the antigen, it is possible to retain the binding of the antibody to its cognate antigen while gain the new functionality of Vm24, which affords a new bispecific antibody by design. To this end, Vm24 was grafted into heavy chain variable region or light chain CDRs of the integrin β7 antibody, or their vicinity, through a coiled-coil-GS linker peptide.

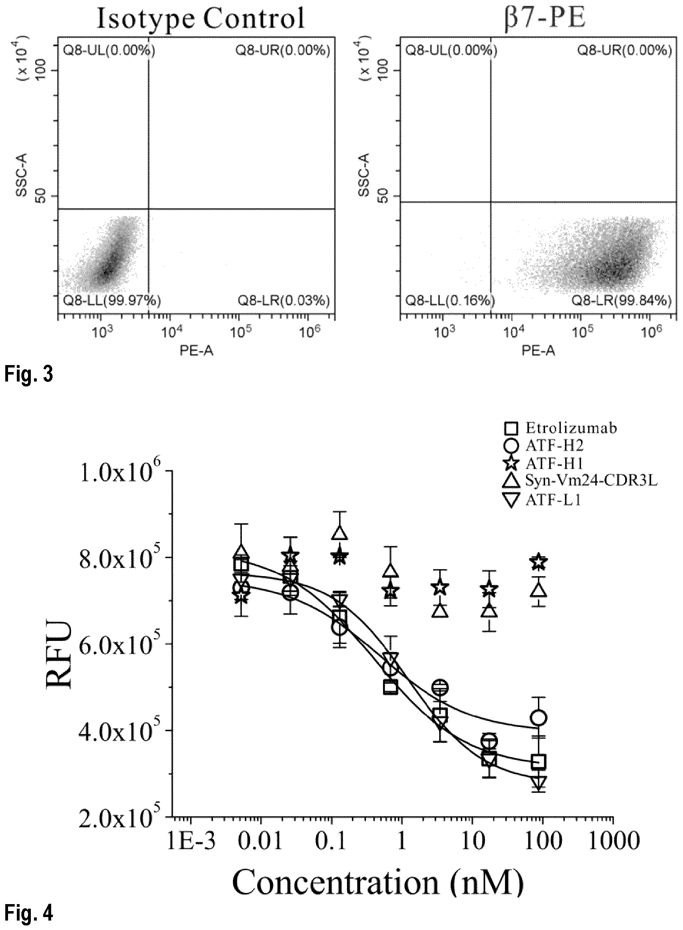

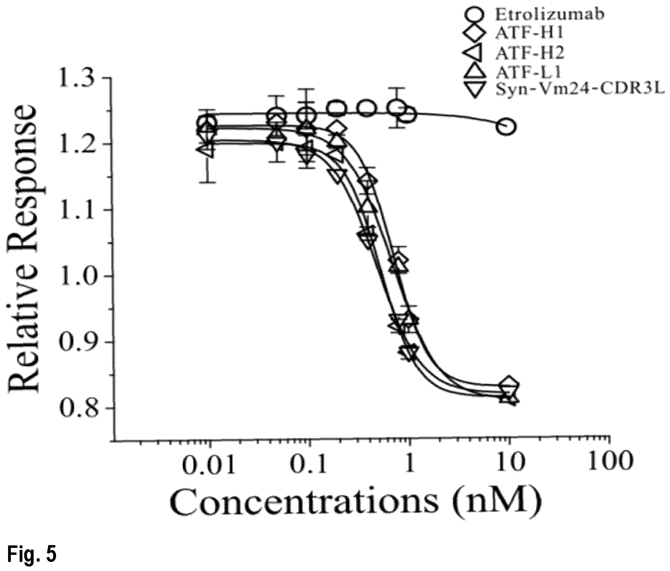

In a preferred embodiment, Vm24 is grafted between 28S and 32S of the light chain variable region CDR1 of the integrin β7 antibody through the coiled-coil-GS linker peptide. The resulting bispecific molecule ATF-L1 has a weaker affinity (167.10 pM) for integrin β7 than that of the integrin β7 antibody (17.57pM), and similar affinity for Kv1.3 as that of Syn-Vm24-LCDR3. In a further embodiment, the inhibitory effect of ATF-H1 on the adhesion of β7-high T cells to MAdCAM-1 is similar to that of the integrin β7 antibody (IC50: 1.09nM vs. 0.42 nM) (Figure 4), while the inhibitory effect on Kv1.3 current is similar to that of Syn-vm24-LCDR3 (Figure 5).

In a preferred embodiment, Vm24 is grafted between the last 26S of the heavy chain variable region FR1 and 32N of the heavy chain variable region CDR1 of the integrin β7 antibody through a coiled-coil-GS linker peptide. The affinity of the resulting bispecific molecule ATF-H1 with integrin β7 significantly reduced compared to the integrin β7 antibody, and its affinity with Kv1.3 is comparable to Syn-Vm24-LCDR3, a fusion protein reported in literature (PNAS 2016,113(41): 11501-11506)(IC50: 0.72 nM vs. 0.49 nM). In further embodiments, ATF-H1 shows little inhibitory effect on the adhesion of β7high T cells to MAdCAM-1 (Figure 4) while displaying similar inhibitory effect on Kv1.3 current to that of Syn-Vm24-LCDR3 (Figure 5)..

In a preferred embodiment, Vm24 is grafted between 60N and 65R of the heavy chain variable region FR3 of the integrin β7 antibody through a coiled-coil-GS linker peptide. The resulting bispecific molecule ATF-H2 has the similar affinity for integrin β7 to that of the integrin β7 antibody (IC50: 19.25 pM vs. 17.57 pM), and also has similar affinity for Kv1.3 to that of Syn-Vm24-LCDR3 reported in literature (PNAS 2016,113(41):11501-11506). In further embodiments, ATF-H1 shows a similar inhibitory effect on the adhesion of β7^{high} T cells to MAdCAM-1 to that of the integrin β7 antibody (IC50: 1.09nM vs 0.42 nM)(Figure 4).

In a preferred embodiment, Vm24 is grafted between 97R and 102G of the heavy chain variable region CDR3 of the integrin β7 antibody through a coiled-coil-GS linker peptide. The resulting bispecific molecule ATF-H3 has the similar affinity for integrin β7 to that of the integrin β7 antibody (IC50: 13.88pM vs. 17.57 pM), and also has similar affinity for Kv1.3 to that of Syn-Vm24-LCDR3 reported in literature (PNAS 2016,113(41):11501-11506).

In a preferred embodiment, Vm24 is grafted between 56S and 60S of the light chain variable region FR3 of the integrin β7 antibody through a coiled-coil-GS linker peptide. The resulting bispecific molecule ATF-L2 has a slightly weaker affinity to integrin β7 than that of the integrin β7 antibody (35.9pM vs. 17.57 pM) and a similar affinity for Kv1.3 to that of Syn-Vm24-LCDR3 reported in literature (PNAS 2016,113(41):11501-11506).

In a preferred embodiment, Vm24 is grafted between 91G and 96N of the light chain variable region CDR3 of the integrin β7 antibody through a coiled-coil-GS linker peptide. The resulting bispecific molecule ATF-L3 has a weaker affinity to integrin β7 than that of the integrin β7 antibody (40.82pM vs. 17.57pM), and a similar affinity for Kv1.3 to that of Syn-Vm24-LCDR3 reported in literature (PNAS 2016,113(41):11501-11506).

### EXAMPLES

### 1. Design and Construction of bispecific binding Molecules

Vm24 is a peptide composed of 36 amino acid residues, with a molecular weight of 3864 Da. The amino acid sequence is: AAAISCVGSPECPPKCRAQGCKNGKCMNRKCKCYYC (SEQ ID NO: 9), and the DNA sequence is:

gccgctgcaatctcctgcgtcggcagccccgaatgtcctcccaagtgccgggctcagggatgcaagaacggcaagtgtatgaaccggaagtgcaagtgcta
ctattgc (SEQ ID NO: 26).

The DNA sequence of complementarity-determining region 1 of Etrolizumab light chain (LCDR1) is gag age gtg gac gac ctg, corresponding to the amino acid sequence 27ESVDDL32. The LCDR2 DNA sequence is aag tac gcc age cag, with corresponding amino acid sequence 49KYASQ53. The LCDR3 DNA sequence is cag cag ggc aac age ctg ccc aac acc, with corresponding amino acid sequence 89QQGNSLPNT97. The DNA sequence of complementarity-determining region 1 of Etrolizumab heavy chain (HCDR1) is TTC TTC ATC ACC AAC AAC, with corresponding amino acid sequence 27FFITNN32. The HCDR2 DNA sequence is ATC AGC TAC AGC GGC AGC ACC, with corresponding amino acid sequence 51ISYSGST57. The HCDR3 DNA sequence is GCC CGC ACC GGC AGC AGC GGC TAC TTC GAC, with corresponding amino acid sequence 96ARTGSSGYFD105.

Vm24 is grafted into CDRs of both the light and heavy chains of etrolizumab, with coiled-coil-GS linkers at both ends (upstream amino acid sequence: GGSGAKLAALKAKLAALKGGGGS; downstream amino acid sequence: GGGGSELAALEAELAALEAGGSG) connecting to the antibody amino acid sequences. The resulting sequences of the bispecific binding molecules are as follows:
Etrolizumab HC(SEQ ID NO: 1): 
EVQLVESGGGLVQPGGSLRLSCAASGFFITNNYWGWVRQAPGKGLEWVGYISYSGSTSYNPSLKSRFTIS
RDTSKNTFYLQMNSLRAEDTAVYYCARTGSSGYFDFWGQGTLVTVSSASTKGPSVFPLAPSSKSTSGGTA
ALGCLVKDYFPEPVTVSWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDVSHEDPEVKFNWYVDG
VEVHNAKTKPREEQYASTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDSDGSFFLYSKLTVDKSRWQQG
NVFSCSVMHEALHNHYTQKSLSLSPGK
Etrolizumab LC(SEQ ID NO: 2): 
DIQMTQSPSSLSASVGDRVTITCRASESVDDLLHWYQQKPGKAPKLLIKYASQSISGVPSRFSGSGSGTDFT
LTISSLQPEDFATYYCQQGNSLPNTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
C
 
ATF-L1 LC(SEQ ID NO: 3):
DIQMTQSPSSLSASVGDRVTITCRASESGGSGAKLAALKAKLAALKGGGGSAAAISCVGSPECPPKCRAQ
GCKNGKCMNRKCKCYYCGGGGSELAALEAELAALEAGGSGLLHWYQQKPGKAPKLLIKYASQSISGVP
SRFSGSGSGTDFTLTISSLQPEDFATYYCQQGNSLPNTFGQGTKVEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC
ATF-L2 LC(SEQ ID NO: 4): 
DIQMTQSPSSLSASVGDRVTITCRASESVDDLLHWYQQKPGKAPKLLIKYASQSISGGSGAKLAALKAKL
AALKGGGGSAAAISCVGSPECPPKCRAQGCKNGKCMNRKCKCYYCGGGGSELAALEAELAALEAGGSG
 
SRFSGSGSGTDFTLTISSLQPEDFATYYCQQGNSLPNTFGQGTKVEIKRTVAAPSVFIFPPSDEQLKSGTASV
VCLLNNFYPREAKVQWKVDNALQSGNSQESVTEQDSKDSTYSLSSTLTLSKADYEKHKVYACEVTHQG
LSSPVTKSFNRGEC
ATF-L3 LC(SEQ ID NO: 5): 
DIQMTQSPSSLSASVGDRVTITCRASESVDDLLHWYQQKPGKAPKLLIKYASQSISGVPSRFSGSGSGTDFT
LTISSLQPEDFATYYCQQGGGSGAKLAALKAKLAALKGGGGSAAAISCVGSPECPPKCRAQGCKNGKCM
NRKCKCYYCGGGGSELAALEAELAALEAGGSGNTFGQGTKVEIKRTVAAPSVFIFPPSDEQLKSGTASVV
CLLNNFYPREAKVQWKVDNALQSGNSQESVTEQDSKDSTYSLSSTLTLSKADYEKHKVYACEVTHQGLS
SPVTKSFNRGEC
ATF-H1 HC(SEQ ID NO: 6): 
EVQLVESGGGLVQPGGSLRLSCAASGGGSGAKLAALKAKLAALKGGGGSAAISCVGSPECPPKCRAQG
CKNGKCMNRKCKCYYCGGGGSELAALEAELAALEAGGSGYWGWVRQAPGKGLEWVGYISYSGSTSY
NPSLKSRFTISRDTSKNTFYLQMNSLRAEDTAVYYCARTGSSGYFDFWGQGTLVTVSSASTKGPSVFPLAP
SSKSTSGGTAALGCLVKDYFPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISRTPEVTCVVVDVSHEDPEV
KFNWYVDGVEVHNAKTKPREEQYASTYRVVSVLTVLHQDWLNGKEYKCKVSNKALPAPIEKTISKAKG
QPREPQVYTLPPSREEMTKNQVSLTCLVKGFYPSDIAVEWESNGQPENNYKTTPPVLDSDGSFFLYSKLTV
DKSRWQQGNVFSCSVMHEALHNHYTQKSLSLSPGK
ATF-H2 HC(SEQ ID NO: 7): 
EVQLVESGGGLVQPGGSLRLSCAASGFFITNNYWGWVRQAPGKGLEWVGYISYSGSTSYNGGSGAKLA
ALKAKLAALKGGGGSAAAISCVGSPECPPKCRAQGCKNGKCMNRKCKCYYCGGGGSELAALEAELAA
LEAGGSGSRFTISRDTSKNTFYLQMNSLRAEDTAVYYCARTGSSGYFDFWGQGTLVTVSSASTKGPSVFP
LAPSSKSTSGGTAALGCLVKDYFPEPVTVSWNSGALTSGVHTFPAVLQSSGLYSLSSVVTVPSSSLGTQTYI
CNVNHKPSNTKVDKKVEPKSCDKTHTCPPCPAPELLGGPSVFLFPPKPKDTLMISRTPEVTCVVVDVSHE
DPEVKFNWYVDGVEVHNAKTKPREEQYASTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPPVLDSDGSFFLY
SKLTVDKSRWQQGNVFSCSVMHEALHNHYTQKSLSLSPGK
ATF-H3 HC(SEQ ID NO: 8): 
EVQLVESGGGLVQPGGSLRLSCAASGFFITNNYWGWVRQAPGKGLEWVGYISYSGSTSYNPSLKSRFTIS
RDTSKNTFYLQMNSLRAEDTAVYYCARGGSGAKLAALKAKLAALKGGGGSAAAISCVGSPECPPKCRA
QGCKNGKCMNRKCKCYYCGGGGSELAALEAELAALEAGGSGGYFDFWGQGTLVTVSSASTKGPSVFPL
APSSKSTSGGTAALGCLVKDYFPEPVTVSWNSGALTSGVHTFPAVLQSSGLYSLSSVVTVPSSSLGTQTYIC
NVNHKPSNTKVDKKVEPKSCDKTHTCPPCPAPELLGGPSVFLFPPKPKDTLMISRTPEVTCVVVDVSHED
PEVKFNWYVDGVEVHNAKTKPREEQYASTYRVVSVLTVLHQDWLNGKEYKCKVSNKALPAPIEKTISK
AKGQPREPQVYTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK
Vm24(SEQ ID NO: 9): AAAISCVGSPECPPKCRAQGCKNGKCMNRKCKCYYC
LCDR1(SEQ ID NO: 10): ²⁷ESVDDL³²
LCDR2(SEQ ID NO: 11): ⁴⁹KYASQ⁵³
LCDR3(SEQ ID NO: 12): ⁸⁹QQGNSLPNT⁹⁷
HCDR1(SEQ ID NO: 13): ²⁷FFITNN³²
HCDR2(SEQ ID NO: 14): ⁵¹ISYSGST⁵⁷
HCDR3(SEQ ID NO: 15): ⁹⁶ARTGSSGYFD¹⁰⁵
Linker 1: GGSGAKLAALKAKLAALKGGGGS(SEQ ID NO: 16)
Linker2: GGGGSELAALEAELAALEAGGSG(SEQ ID NO: 17)
Etrolizumab HC(SEQ ID NO: 18) Nucleotide sequence: 
GAGGTGCAGCTGGTGGAGAGCGGCGGCGGCCTGGTGCAGCCCGGCGGCAGCCTGCGCCTGAGCTGC
GCCGCCAGCGGCTTCTTCATCACCAACAACTACTGGGGCTGGGTGCGCCAGGCCCCCGGCAAGGGCC
TGGAGTGGGTGGGCTACATCAGCTACAGCGGCAGCACCAGCTACAACCCCAGCCTGAAGAGCCGCTT
CACCATCAGCCGCGACACCAGCAAGAACACCTTCTACCTGCAGATGAACAGCCTGCGCGCCGAGGAC
ACCGCCGTGTACTACTGCGCCCGCACCGGCAGCAGCGGCTACTTCGACTTCTGGGGCCAGGGCACCC
TGGTGACCGTGAGCAGCGCCTCCACCAAGGGCCCATCGGTCTTCCCCCTGGCACCCTCCTCCAAGAG
CACCTCTGGGGGCACAGCGGCCCTGGGCTGCCTGGTCAAGGACTACTTCCCCGAACCGGTGACGGTG
TCGTGGAACTCAGGCGCCCTGACCAGCGGCGTGCACACCTTCCCGGCTGTCCTACAGTCCTCAGGAC
TCTACTCCCTCAGCAGCGTGGTGACTGTGCCCTCTAGCAGCTTGGGCACCCAGACCTACATCTGCAAC
GTGAATCACAAGCCCAGCAACACCAAGGTGGACAAGAAAGTTGAACCCAAATCTTGCGACAAAACT
CACACATGCCCACCGTGCCCAGCACCTGAACTCCTGGGGGGACCGTCAGTCTTCCTCTTCCCCCCAA
AACCCAAGGACACCCTCATGATCTCCCGGACCCCTGAGGTCACATGCGTGGTGGTGGACGTGAGCCA
CGAAGACCCTGAGGTCAAGTTCAACTGGTACGTGGACGGCGTGGAGGTGCATAATGCCAAGACAAA
GCCGCGGGAGGAGCAGTACGCCAGCACGTACCGTGTGGTCAGCGTCCTCACCGTCCTGCACCAGGA
CTGGCTGAATGGCAAGGAGTACAAGTGCAAGGTCTCCAACAAAGCCCTCCCAGCCCCCATAGAGAA
AACCATCTCCAAAGCCAAAGGGCAGCCCCGAGAACCACAGGTGTACACCCTGCCCCCATCCCGGGA
GGAGATGACCAAGAACCAGGTCAGCCTGACCTGCCTGGTCAAAGGCTTCTATCCCAGCGACATCGCC
GTGGAGTGGGAGAGCAATGGGCAGCCGGAGAACAACTACAAGACCACGCCTCCCGTGCTGGACTCC
GACGGCTCCTTCTTCCTCTACAGCAAGCTCACCGTGGACAAGAGCAGGTGGCAGCAGGGGAACGTCT
TCTCATGCTCCGTGATGCATGAGGCTCTGCACAACCACTACACGCAGAAGAGCCTCTCCCTGTCTCCG
GGTAAA
Etrolizumab LC(SEQ ID NO: 19) Nucleotide sequence: 
GACATCCAGATGACCCAGAGCCCCAGCAGCCTGAGCGCCAGCGTGGGCGACCGCGTGACCATCACCT
GCCGCGCCAGCGAGAGCGTGGACGACCTGCTGCACTGGTACCAGCAGAAGCCCGGCAAGGCCCCCA
AGCTGCTGATCAAGTACGCCAGCCAGAGCATCAGCGGCGTGCCCAGCCGCTTCAGCGGCAGCGGCA
GCGGCACCGACTTCACCCTGACCATCAGCAGCCTGCAGCCCGAGGACTTCGCCACCTACTACTGCCA
GCAGGGCAACAGCCTGCCCAACACCTTCGGCCAGGGCACCAAGGTGGAGATCAAGCGCACAGTGGC
AGCCCCCAGCGTCTTCATTTTTCCCCCTTCCGATGAACAGCTGAAGTCCGGCACTGCTTCTGTGGTCT
GTCTGCTGAACAATTTCTATCCCAGAGAGGCCAAGGTGCAGTGGAAAGTGGACAACGCTCTGCAGTC
CGGCAACAGCCAGGAGAGTGTGACCGAACAGGATAGTAAGGACAGCACATATTCTCTGTCTAGTACC
CTGACACTGAGTAAGGCAGATTACGAGAAGCACAAAGTGTATGCCTGCGAAGTCACTCATCAGGGAC
TGTCAAGCCCCGTGACCAAGAGCTTCAACCGGGGCGAGTGT
ATF-L1 LC(SEQ ID NO: 20) Nucleotide sequence: 
GACATCCAGATGACCCAGAGCCCCAGCAGCCTGAGCGCCAGCGTGGGCGACCGCGTGACCATCACCT
GCCGCGCCAGCGAGAGCGGCGGAAGCGGAGCAAAGCTCGCCGCACTGAAAGCCAAGCTGGCCGCT
CTGAAGGGAGGTGGCGGGAGCGCCGCTGCAATCTCCTGCGTCGGCAGCCCCGAATGTCCTCCCAAGT
GCCGGGCTCAGGGATGCAAGAACGGCAAGTGTATGAACCGGAAGTGCAAGTGCTACTATTGCGGCGG
AGGTGGGAGTGAACTGGCCGCACTGGAAGCTGAGCTGGCTGCCCTCGAAGCTGGAGGCTCTGGACT
GCTGCACTGGTACCAGCAGAAGCCCGGCAAGGCCCCCAAGCTGCTGATCAAGTACGCCAGCCAGAG
CATCAGCGGCGTGCCCAGCCGCTTCAGCGGCAGCGGCAGCGGCACCGACTTCACCCTGACCATCAGC
 
AGCCTGCAGCCCGAGGACTTCGCCACCTACTACTGCCAGCAGGGCAACAGCCTGCCCAACACCTTCG
GCCAGGGCACCAAGGTGGAGATCAAGCGCACAGTGGCAGCCCCCAGCGTCTTCATTTTTCCCCCTTC
CGATGAACAGCTGAAGTCCGGCACTGCTTCTGTGGTCTGTCTGCTGAACAATTTCTATCCCAGAGAGG
CCAAGGTGCAGTGGAAAGTGGACAACGCTCTGCAGTCCGGCAACAGCCAGGAGAGTGTGACCGAAC
AGGATAGTAAGGACAGCACATATTCTCTGTCTAGTACCCTGACACTGAGTAAGGCAGATTACGAGAAG
CACAAAGTGTATGCCTGCGAAGTCACTCATCAGGGACTGTCAAGCCCCGTGACCAAGAGCTTCAACC
GGGGCGAGTGT
ATF-L2 LC(SEQ ID NO: 21) Nucleotide sequence: 
GACATCCAGATGACCCAGAGCCCCAGCAGCCTGAGCGCCAGCGTGGGCGACCGCGTGACCATCACCT
GCCGCGCCAGCGAGAGCGTGGACGACCTGCTGCACTGGTACCAGCAGAAGCCCGGCAAGGCCCCCA
AGCTGCTGATCAAGTACGCCAGCCAGAGCATCAGCGGCGGAAGCGGAGCAAAGCTCGCCGCACTGA
AAGCCAAGCTGGCCGCTCTGAAGGGAGGTGGCGGGAGCGCCGCTGCAATCTCCTGCGTCGGCAGCC
CCGAATGTCCTCCCAAGTGCCGGGCTCAGGGATGCAAGAACGGCAAGTGTATGAACCGGAAGTGCA
AGTGCTACTATTGCGGCGGAGGTGGGAGTGAACTGGCCGCACTGGAAGCTGAGCTGGCTGCCCTCGA
AGCTGGAGGCTCTGGAAGCCGCTTCAGCGGCAGCGGCAGCGGCACCGACTTCACCCTGACCATCAG
CAGCCTGCAGCCCGAGGACTTCGCCACCTACTACTGCCAGCAGGGCAACAGCCTGCCCAACACCTTC
GGCCAGGGCACCAAGGTGGAGATCAAGCGCACAGTGGCAGCCCCCAGCGTCTTCATTTTTCCCCCTT
CCGATGAACAGCTGAAGTCCGGCACTGCTTCTGTGGTCTGTCTGCTGAACAATTTCTATCCCAGAGAG
GCCAAGGTGCAGTGGAAAGTGGACAACGCTCTGCAGTCCGGCAACAGCCAGGAGAGTGTGACCGAA
CAGGATAGTAAGGACAGCACATATTCTCTGTCTAGTACCCTGACACTGAGTAAGGCAGATTACGAGAA
GCACAAAGTGTATGCCTGCGAAGTCACTCATCAGGGACTGTCAAGCCCCGTGACCAAGAGCTTCAAC
CGGGGCGAGTGT
ATF-L3 LC(SEQ ID NO: 22) Nucleotide sequence: 
GACATCCAGATGACCCAGAGCCCCAGCAGCCTGAGCGCCAGCGTGGGCGACCGCGTGACCATCACCT
GCCGCGCCAGCGAGAGCGTGGACGACCTGCTGCACTGGTACCAGCAGAAGCCCGGCAAGGCCCCCA
AGCTGCTGATCAAGTACGCCAGCCAGAGCATCAGCGGCGTGCCCAGCCGCTTCAGCGGCAGCGGCA
GCGGCACCGACTTCACCCTGACCATCAGCAGCCTGCAGCCCGAGGACTTCGCCACCTACTACTGCCA
GCAGGGCGGCGGAAGCGGAGCAAAGCTCGCCGCACTGAAAGCCAAGCTGGCCGCTCTGAAGGGAG
GTGGCGGGAGCGCCGCTGCAATCTCCTGCGTCGGCAGCCCCGAATGTCCTCCCAAGTGCCGGGCTCA
GGGATGCAAGAACGGCAAGTGTATGAACCGGAAGTGCAAGTGCTACTATTGCGGCGGAGGTGGGAG
TGAACTGGCCGCACTGGAAGCTGAGCTGGCTGCCCTCGAAGCTGGAGGCTCTGGAAACACCTTCGG
CCAGGGCACCAAGGTGGAGATCAAGCGCACAGTGGCAGCCCCCAGCGTCTTCATTTTTCCCCCTTCC
GATGAACAGCTGAAGTCCGGCACTGCTTCTGTGGTCTGTCTGCTGAACAATTTCTATCCCAGAGAGGC
CAAGGTGCAGTGGAAAGTGGACAACGCTCTGCAGTCCGGCAACAGCCAGGAGAGTGTGACCGAACA
GGATAGTAAGGACAGCACATATTCTCTGTCTAGTACCCTGACACTGAGTAAGGCAGATTACGAGAAGC
ACAAAGTGTATGCCTGCGAAGTCACTCATCAGGGACTGTCAAGCCCCGTGACCAAGAGCTTCAACCG
GGGCGAGTGT
ATF-H1 HC(SEQ ID NO: 23) Nucleotide sequence: 
GAGGTGCAGCTGGTGGAGAGCGGCGGCGGCCTGGTGCAGCCCGGCGGCAGCCTGCGCCTGAGCTGC
GCCGCCAGCGGCGGCGGAAGCGGAGCAAAGCTCGCCGCACTGAAAGCCAAGCTGGCCGCTCTGAAG
GGAGGTGGCGGGAGCGCCGCTGCAATCTCCTGCGTCGGCAGCCCCGAATGTCCTCCCAAGTGCCGGG
CTCAGGGATGCAAGAACGGCAAGTGTATGAACCGGAAGTGCAAGTGCTACTATTGCGGCGGAGGTGG
GAGTGAACTGGCCGCACTGGAAGCTGAGCTGGCTGCCCTCGAAGCTGGAGGCTCTGGATACTGGGG
CTGGGTGCGCCAGGCCCCCGGCAAGGGCCTGGAGTGGGTGGGCTACATCAGCTACAGCGGCAGCAC
CAGCTACAACCCCAGCCTGAAGAGCCGCTTCACCATCAGCCGCGACACCAGCAAGAACACCTTCTAC
 
CTGCAGATGAACAGCCTGCGCGCCGAGGACACCGCCGTGTACTACTGCGCCCGCACCGGCAGCAGC
GGCTACTTCGACTTCTGGGGCCAGGGCACCCTGGTGACCGTGAGCAGCGCCTCCACCAAGGGCCCAT
CGGTCTTCCCCCTGGCACCCTCCTCCAAGAGCACCTCTGGGGGCACAGCGGCCCTGGGCTGCCTGGT
CAAGGACTACTTCCCCGAACCGGTGACGGTGTCGTGGAACTCAGGCGCCCTGACCAGCGGCGTGCA
CACCTTCCCGGCTGTCCTACAGTCCTCAGGACTCTACTCCCTCAGCAGCGTGGTGACTGTGCCCTCTA
GCAGCTTGGGCACCCAGACCTACATCTGCAACGTGAATCACAAGCCCAGCAACACCAAGGTGGACA
AGAAAGTTGAACCCAAATCTTGCGACAAAACTCACACATGCCCACCGTGCCCAGCACCTGAACTCCT
GGGGGGACCGTCAGTCTTCCTCTTCCCCCCAAAACCCAAGGACACCCTCATGATCTCCCGGACCCCT
GAGGTCACATGCGTGGTGGTGGACGTGAGCCACGAAGACCCTGAGGTCAAGTTCAACTGGTACGTG
GACGGCGTGGAGGTGCATAATGCCAAGACAAAGCCGCGGGAGGAGCAGTACGCCAGCACGTACCGT
GTGGTCAGCGTCCTCACCGTCCTGCACCAGGACTGGCTGAATGGCAAGGAGTACAAGTGCAAGGTCT
CCAACAAAGCCCTCCCAGCCCCCATAGAGAAAACCATCTCCAAAGCCAAAGGGCAGCCCCGAGAAC
CACAGGTGTACACCCTGCCCCCATCCCGGGAGGAGATGACCAAGAACCAGGTCAGCCTGACCTGCCT
GGTCAAAGGCTTCTATCCCAGCGACATCGCCGTGGAGTGGGAGAGCAATGGGCAGCCGGAGAACAA
CTACAAGACCACGCCTCCCGTGCTGGACTCCGACGGCTCCTTCTTCCTCTACAGCAAGCTCACCGTGG
ACAAGAGCAGGTGGCAGCAGGGGAACGTCTTCTCATGCTCCGTGATGCATGAGGCTCTGCACAACCA
CTACACGCAGAAGAGCCTCTCCCTGTCTCCGGGTAAA
ATF-H2 HC(SEQ ID NO: 24) Nucleotide sequence: 
GAGGTGCAGCTGGTGGAGAGCGGCGGCGGCCTGGTGCAGCCCGGCGGCAGCCTGCGCCTGAGCTGC
GCCGCCAGCGGCTTCTTCATCACCAACAACTACTGGGGCTGGGTGCGCCAGGCCCCCGGCAAGGGCC
TGGAGTGGGTGGGCTACATCAGCTACAGCGGCAGCACCAGCTACAACGGCGGAAGCGGAGCAAAGC
TCGCCGCACTGAAAGCCAAGCTGGCCGCTCTGAAGGGAGGTGGCGGGAGCGCCGCTGCAATCTCCT
GCGTCGGCAGCCCCGAATGTCCTCCCAAGTGCCGGGCTCAGGGATGCAAGAACGGCAAGTGTATGAA
CCGGAAGTGCAAGTGCTACTATTGCGGCGGAGGTGGGAGTGAACTGGCCGCACTGGAAGCTGAGCT
GGCTGCCCTCGAAGCTGGAGGCTCTGGAAGCCGCTTCACCATCAGCCGCGACACCAGCAAGAACAC
CTTCTACCTGCAGATGAACAGCCTGCGCGCCGAGGACACCGCCGTGTACTACTGCGCCCGCACCGGC
AGCAGCGGCTACTTCGACTTCTGGGGCCAGGGCACCCTGGTGACCGTGAGCAGCGCCTCCACCAAG
GGCCCATCGGTCTTCCCCCTGGCACCCTCCTCCAAGAGCACCTCTGGGGGCACAGCGGCCCTGGGCT
GCCTGGTCAAGGACTACTTCCCCGAACCGGTGACGGTGTCGTGGAACTCAGGCGCCCTGACCAGCGG
CGTGCACACCTTCCCGGCTGTCCTACAGTCCTCAGGACTCTACTCCCTCAGCAGCGTGGTGACTGTGC
CCTCTAGCAGCTTGGGCACCCAGACCTACATCTGCAACGTGAATCACAAGCCCAGCAACACCAAGGT
GGACAAGAAAGTTGAACCCAAATCTTGCGACAAAACTCACACATGCCCACCGTGCCCAGCACCTGA
ACTCCTGGGGGGACCGTCAGTCTTCCTCTTCCCCCCAAAACCCAAGGACACCCTCATGATCTCCCGGA
CCCCTGAGGTCACATGCGTGGTGGTGGACGTGAGCCACGAAGACCCTGAGGTCAAGTTCAACTGGTA
CGTGGACGGCGTGGAGGTGCATAATGCCAAGACAAAGCCGCGGGAGGAGCAGTACGCCAGCACGTA
CCGTGTGGTCAGCGTCCTCACCGTCCTGCACCAGGACTGGCTGAATGGCAAGGAGTACAAGTGCAAG
GTCTCCAACAAAGCCCTCCCAGCCCCCATAGAGAAAACCATCTCCAAAGCCAAAGGGCAGCCCCGA
GAACCACAGGTGTACACCCTGCCCCCATCCCGGGAGGAGATGACCAAGAACCAGGTCAGCCTGACCT
GCCTGGTCAAAGGCTTCTATCCCAGCGACATCGCCGTGGAGTGGGAGAGCAATGGGCAGCCGGAGA
ACAACTACAAGACCACGCCTCCCGTGCTGGACTCCGACGGCTCCTTCTTCCTCTACAGCAAGCTCAC
CGTGGACAAGAGCAGGTGGCAGCAGGGGAACGTCTTCTCATGCTCCGTGATGCATGAGGCTCTGCAC
AACCACTACACGCAGAAGAGCCTCTCCCTGTCTCCGGGTAAA
ATF-H3 HC(SEQ ID NO: 25) Nucleotide sequence: 
GAGGTGCAGCTGGTGGAGAGCGGCGGCGGCCTGGTGCAGCCCGGCGGCAGCCTGCGCCTGAGCTGC
GCCGCCAGCGGCTTCTTCATCACCAACAACTACTGGGGCTGGGTGCGCCAGGCCCCCGGCAAGGGCC
 
TGGAGTGGGTGGGCTACATCAGCTACAGCGGCAGCACCAGCTACAACCCCAGCCTGAAGAGCCGCTT
CACCATCAGCCGCGACACCAGCAAGAACACCTTCTACCTGCAGATGAACAGCCTGCGCGCCGAGGAC
ACCGCCGTGTACTACTGCGCCCGCGGCGGAAGCGGAGCAAAGCTCGCCGCACTGAAAGCCAAGCTG
GCCGCTCTGAAGGGAGGTGGCGGGAGCGCCGCTGCAATCTCCTGCGTCGGCAGCCCCGAATGTCCTC
CCAAGTGCCGGGCTCAGGGATGCAAGAACGGCAAGTGTATGAACCGGAAGTGCAAGTGCTACTATTG
CGGCGGAGGTGGGAGTGAACTGGCCGCACTGGAAGCTGAGCTGGCTGCCCTCGAAGCTGGAGGCTC
TGGAGGCTACTTCGACTTCTGGGGCCAGGGCACCCTGGTGACCGTGAGCAGCGCCTCCACCAAGGGC
CCATCGGTCTTCCCCCTGGCACCCTCCTCCAAGAGCACCTCTGGGGGCACAGCGGCCCTGGGCTGCC
TGGTCAAGGACTACTTCCCCGAACCGGTGACGGTGTCGTGGAACTCAGGCGCCCTGACCAGCGGCGT
GCACACCTTCCCGGCTGTCCTACAGTCCTCAGGACTCTACTCCCTCAGCAGCGTGGTGACTGTGCCCT
CTAGCAGCTTGGGCACCCAGACCTACATCTGCAACGTGAATCACAAGCCCAGCAACACCAAGGTGGA
CAAGAAAGTTGAACCCAAATCTTGCGACAAAACTCACACATGCCCACCGTGCCCAGCACCTGAACTC
CTGGGGGGACCGTCAGTCTTCCTCTTCCCCCCAAAACCCAAGGACACCCTCATGATCTCCCGGACCCC
TGAGGTCACATGCGTGGTGGTGGACGTGAGCCACGAAGACCCTGAGGTCAAGTTCAACTGGTACGTG
GACGGCGTGGAGGTGCATAATGCCAAGACAAAGCCGCGGGAGGAGCAGTACGCCAGCACGTACCGT
GTGGTCAGCGTCCTCACCGTCCTGCACCAGGACTGGCTGAATGGCAAGGAGTACAAGTGCAAGGTCT
CCAACAAAGCCCTCCCAGCCCCCATAGAGAAAACCATCTCCAAAGCCAAAGGGCAGCCCCGAGAAC
CACAGGTGTACACCCTGCCCCCATCCCGGGAGGAGATGACCAAGAACCAGGTCAGCCTGACCTGCCT
GGTCAAAGGCTTCTATCCCAGCGACATCGCCGTGGAGTGGGAGAGCAATGGGCAGCCGGAGAACAA
CTACAAGACCACGCCTCCCGTGCTGGACTCCGACGGCTCCTTCTTCCTCTACAGCAAGCTCACCGTGG
ACAAGAGCAGGTGGCAGCAGGGGAACGTCTTCTCATGCTCCGTGATGCATGAGGCTCTGCACAACCA
CTACACGCAGAAGAGCCTCTCCCTGTCTCCGGGTAAA
Vm24(SEQ ID NO: 26) Nucleotide sequence:

GCCGCTGCAATCTCCTGCGTCGGCAGCCCCGAATGTCCTCCCAAGTGCCGGGCTCAGGGATGCAAGA
ACGGCAAGTGTATGAACCGGAAGTGCAAGTGCTACTATTGC

### 2. Expression and purification of bispecific binding molecules

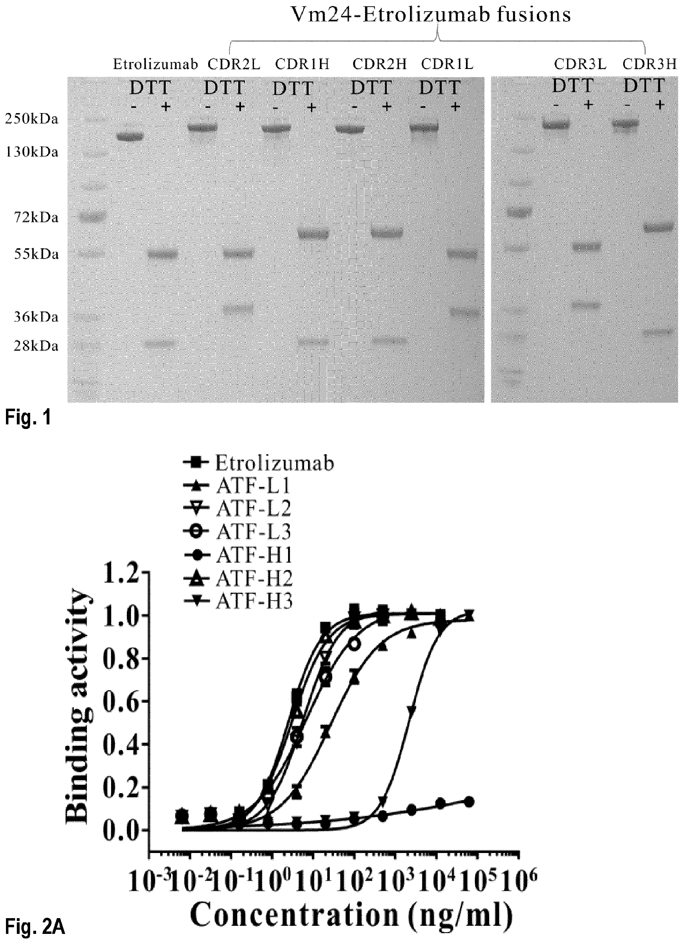

The constructed plasmids of bispecific binding molecules were transiently transfected into HEK293F cells for expression. The expression levels varied from 7 mg/L to 23 mg/L for different constructs. Under reducing agent DTT conditions, the molecular weights of HC and LC of bispecific binding molecules matched with the expectation (for example, when Vm24 is grafted into HC, then the molecular weight of corresponding HC is bigger), with the expression purity exceeding 90% (Figure 1). The proteins were purified by size-exclusion chromatography(SEC), and then applied to binding and cell functional assays. Proteins for animal experiments were further purified with endotoxin removal kit.

### 3. Affinity assay of bispecific binding molecules

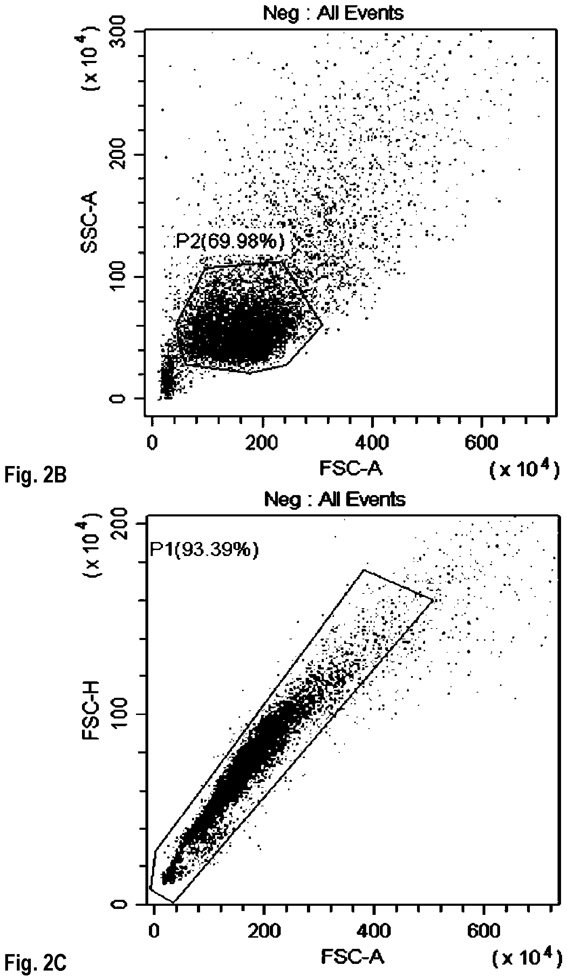

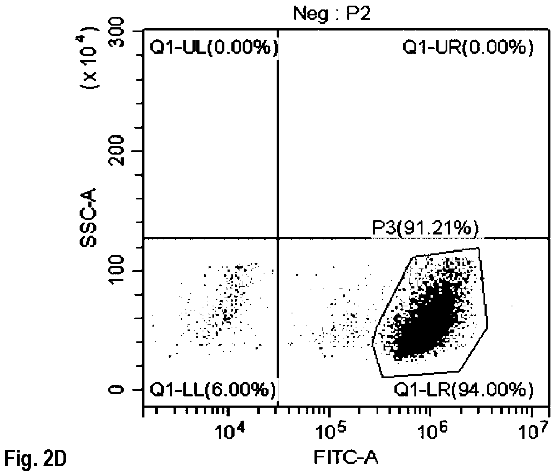

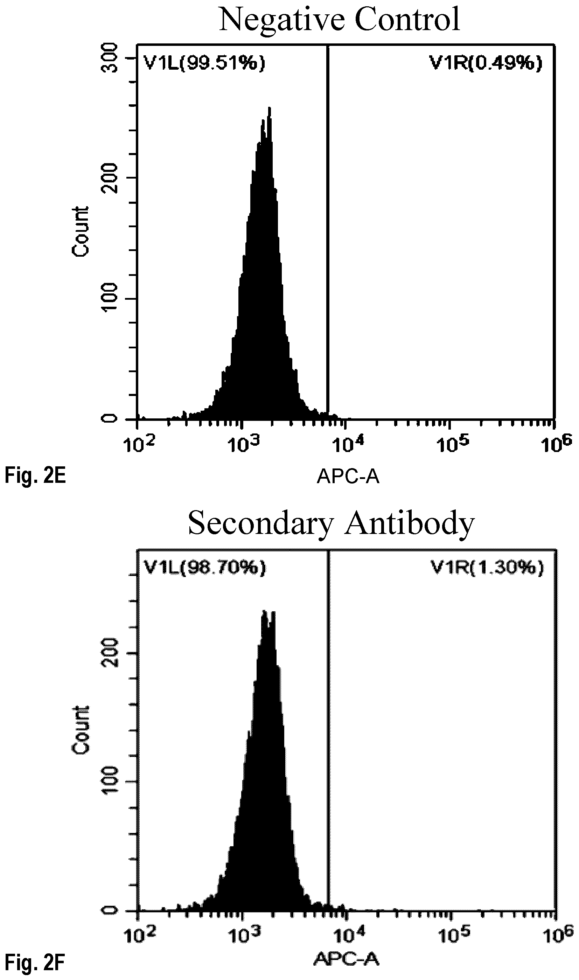

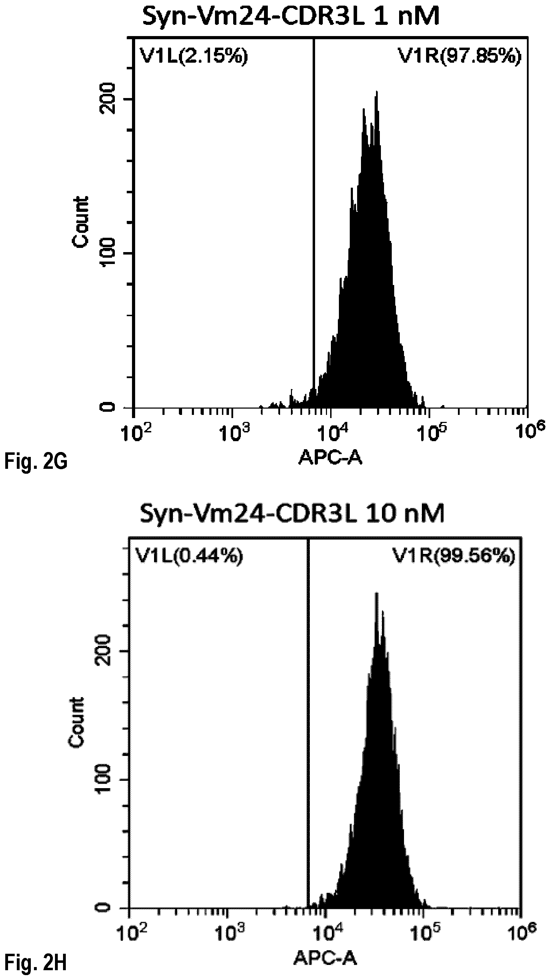

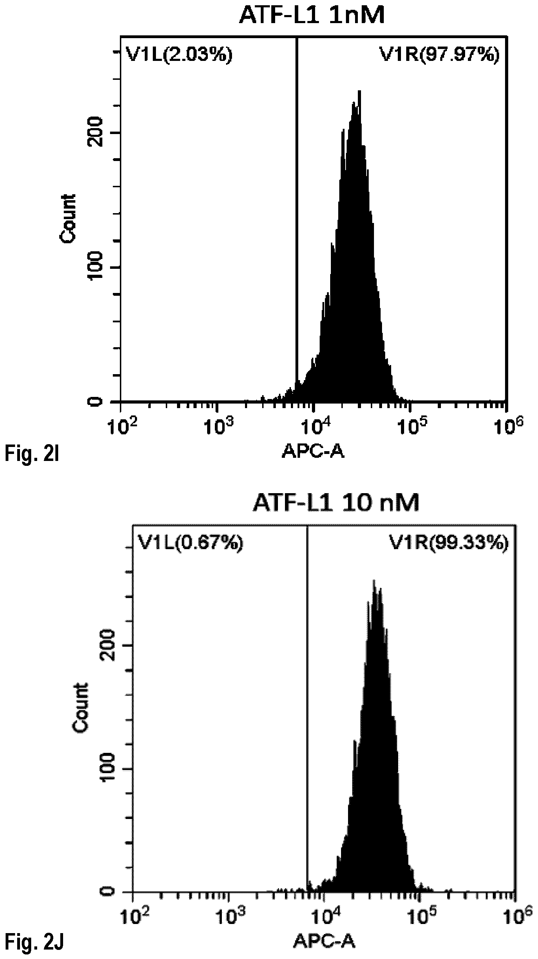

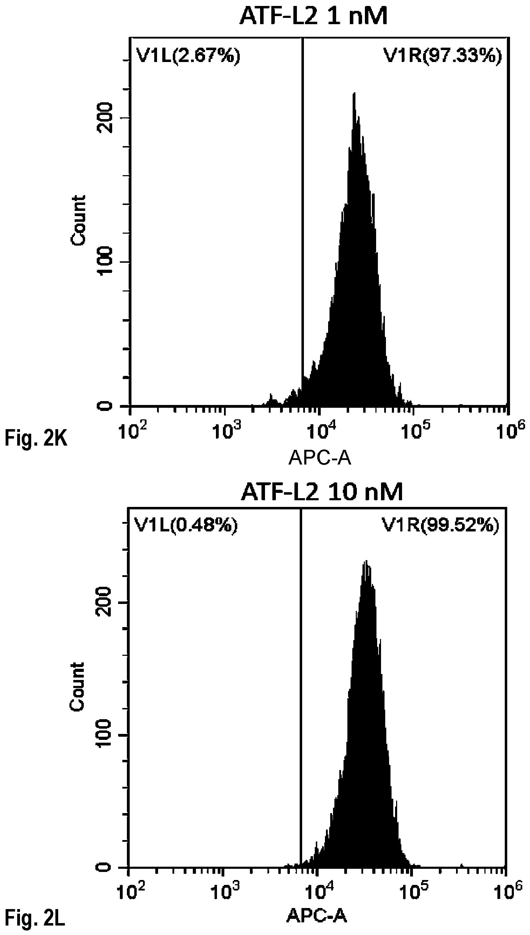

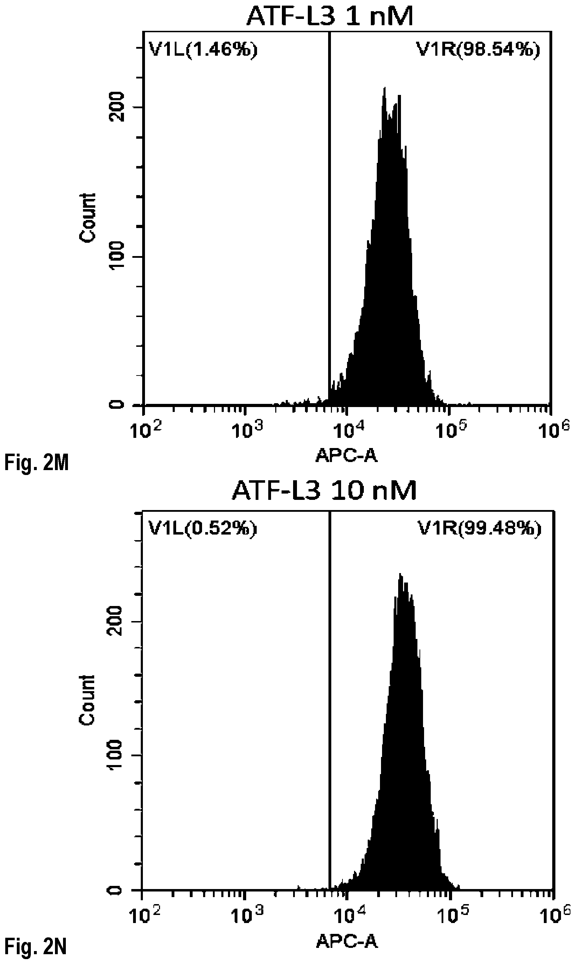

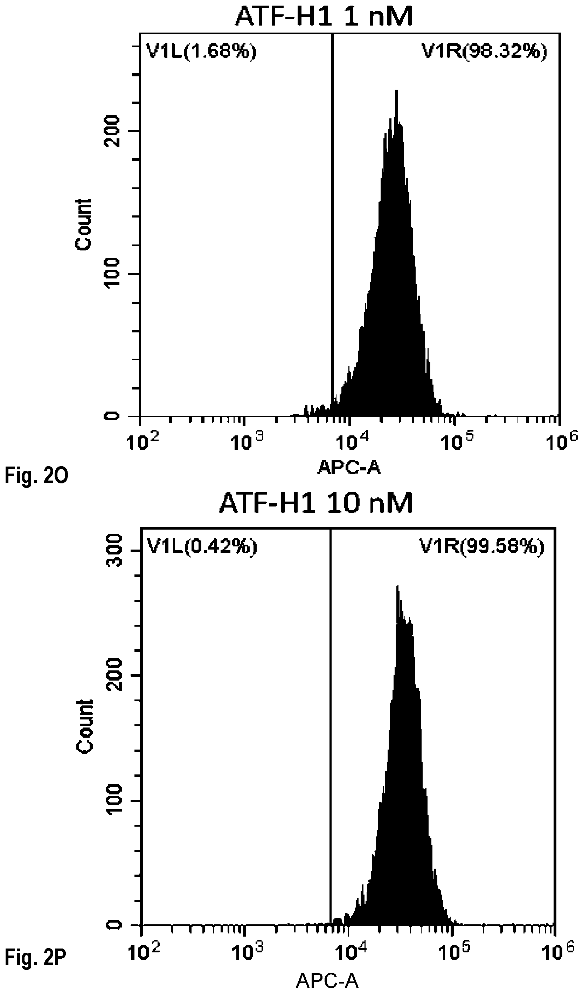

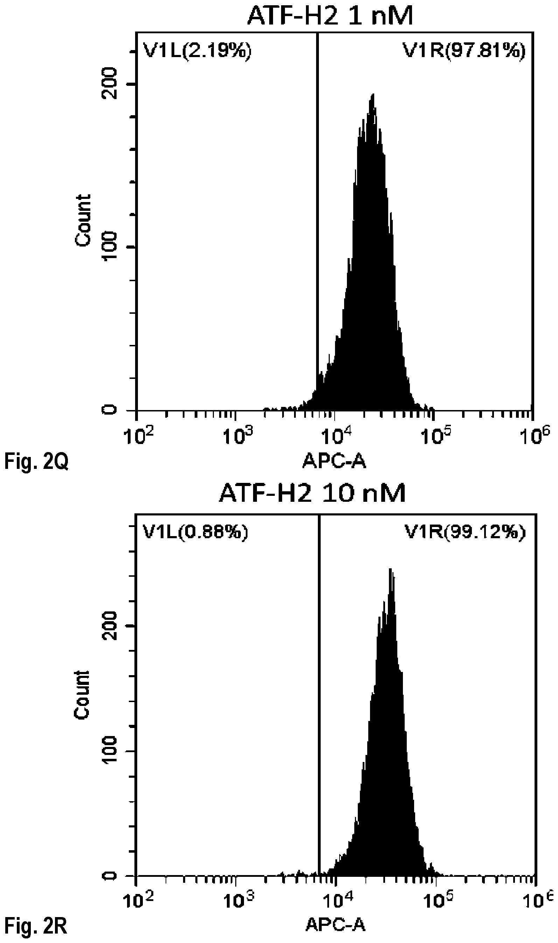

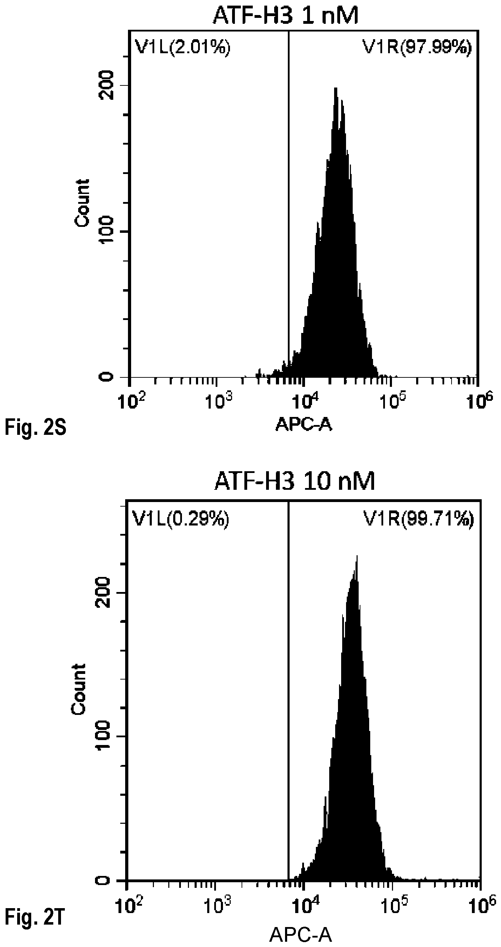

Recombinant protein α4β7 was coated on a 96-well plate at 100 ng per well, and enzyme-linked immunosorbent assay (ELISA) was applied to detect the binding capacity of the bispecific binding molecules. Results showed that the Kd values for α4β7 binding of ATF-H2 (with HCDR2-grafted Vm24), ATF-L2 (with LCDR2-grafted Vm24) and ATF-L3 (with LCDR3-grafted Vm24) were 19.25 ± 1.45 pM, 35.9 ± 2.5 pM, and 40.82 ± 6.6 pM, respectively, closely approaching the value of Kd = 17.57±1.46 pM for etrolizumab. ATF-L1, with LCDR1-grafted Vm24, showed a decreased affinity (Kd = 167.10±16.42 pM) compared to aforementioned three bispecific molecules (ATF-H2, ATF-L2 and ATF-L3). The Kd value of ATF-H3 (with HCDR3-grafted Vm24) was 13.88±1.78 nM, while ATF-H1(with HCDR1-grafted Vm24) showed no binding for α4β7 (Figure 2A). Flow cytometry was applied to detect the binding of the bispecific binding molecules to K v 1.3 ion channel on stable cell line. The results indicated that the bispecific binding molecules showed comparable binding activity to that of Syn-Vm24-LCDR3 reported in previous literature (Wang. R.E. et al. PNAS 2016,113(41): 11501-11506) at both concentrations of 1nM and 10 nM (Figure 2B).

### 4. In vitro activity of bispecific binding molecules

### 4.1 Cell adhesion assay

Human PBMCs were stimulated with PMA (5 ng/ml) and ionomycin (500 ng/ml) for 5-6 hours, and then transferred to normal medium. After 6-7 days, the cells were further stimulated as previously until β7^{high} T cells has been induced (in general, at least 3 stimulation needed) (Figure 3). The cells were used for cell adhesion inhibition experiment.

96-well ELISA plates were coated with recombinant human MAdCAM-1(200ng/well) overnight at 4 °C. The coating solution was removed and the plates were washed for 2-3 times with DPBS. Then, the plates were blocked at 37 °C for 1 h. The β7^{high} PBMCs (2×10⁵ cells/well) were resuspended with detection solution (1 mM Mn²⁺, DMEM, 0.1% BSA, 10 mM HEPES, pH 7), and incubated with different concentrations of the integrin β7 antibody, the bispecific binding molecules and control antibody at 37°C for 30 minutes (2* 105 cells/well in 96-well plate), followed by another 1h incubation to allow cells adhesion. Gently remove the supernatant and wash the plate with DPBS 2-3 times to clean out the cells not attached to the wall. After the last wash,, 100 ul complete medium were added and equilibrated at room temperature for 30 minutes. CellTiter-Glo reagent was then added to each well, shaked and mixed for 2 minutes to lyse the cells. After incubation of the mixture at room temperature for 10 minutes, relative fluorescence units (RFU) was recorded with a microplate reader, and the IC₅₀ was calculated.

Results showed that IC₅₀ values were 0.06 ± 0.02 µg/ml (0.42 nM ± 0.14 nM), 0.072 ± 0.052 µg/ml (0.45 nM ± 0.33 nM) and 0.173 ± 0.023 µg/ml (1.09 nM ± 0.14 nM) for etrolizumab, ATF-H2 and ATF-L1, respectively. ATF-H1 and Syn-Vm24-LCDR3 showed no significant inhibitory effect on the adhesion of β7^{high}T cells to MAdCAM-1 (Figure 4).

### 4.2 Flipr potassium current fluorescence detection

The inhibitory effects of bispecific binding molecules on Kv 1.3 current were assessed using the Flipr potassium current assay kit. The real-time fluorescence signal of Kv1.3 current was recorded by the Flipr high-throughput real-time fluorescence system. The IC₅₀ values were calculated, and the inhibitory effects were compared. The results showed that the IC₅₀ of the Syn-Vm24-LCDR3 we previously constructed was 0.49 ± 0.02nM; the IC₅₀ of ATF-H1, ATF-H2, and ATF-L1 were 0.72 ± 0.05 nM, 0.53 ± 0.02nM, and 0.70 ± 0.06 nM, respectively, while etrolizumab showed no inhibitory effect on the Kv1.3 channel current (Figure 5).

### 5. Selectivity of bispecific binding molecules for Kv1.3 and β7

### 5.1 Generation of Kv1.3^{high} β7^{high} T Cell model

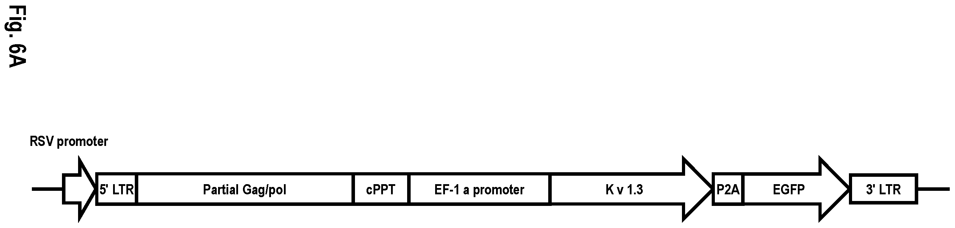

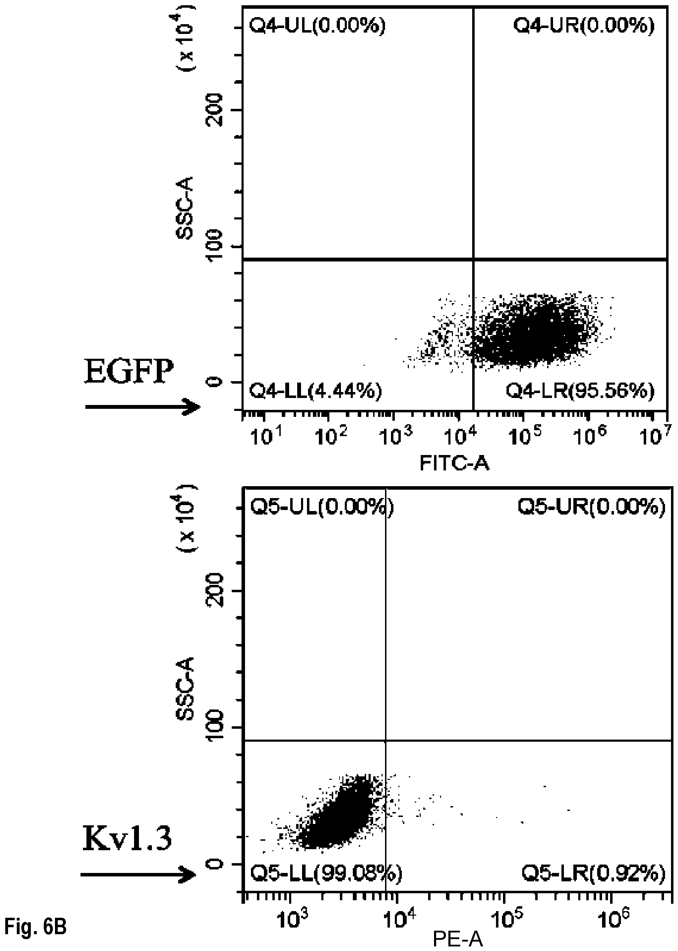

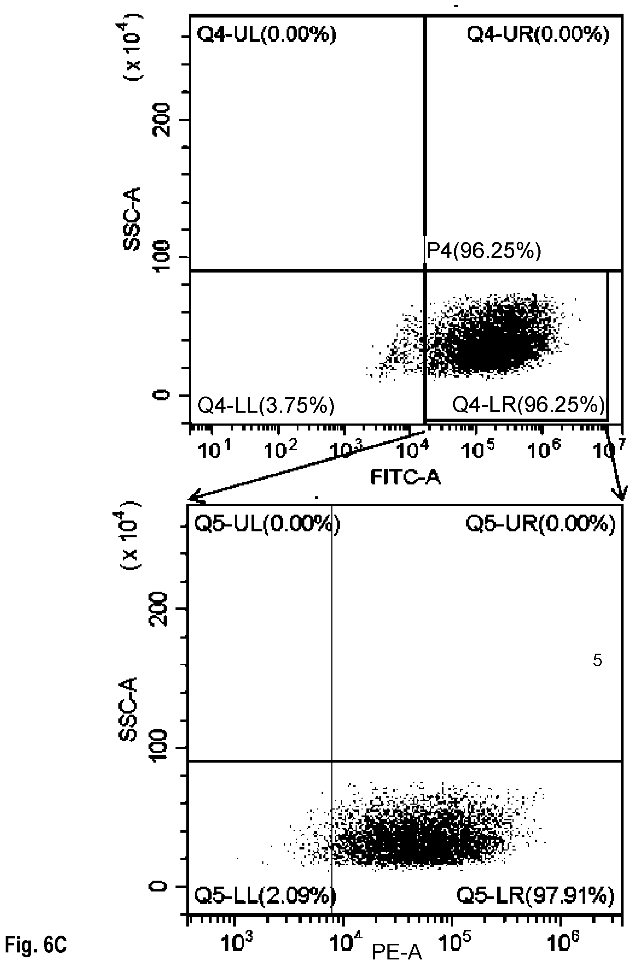

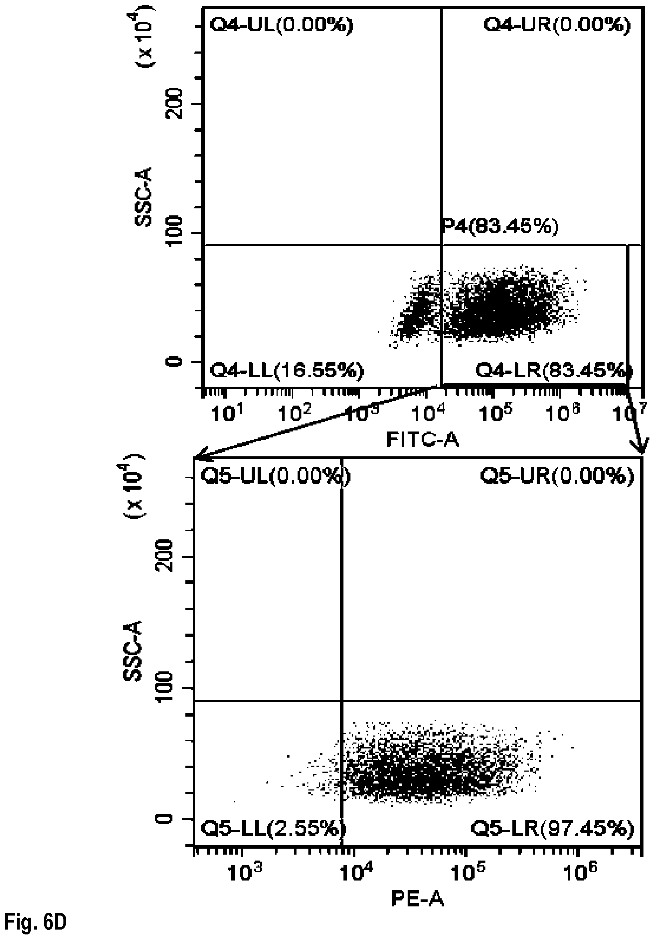

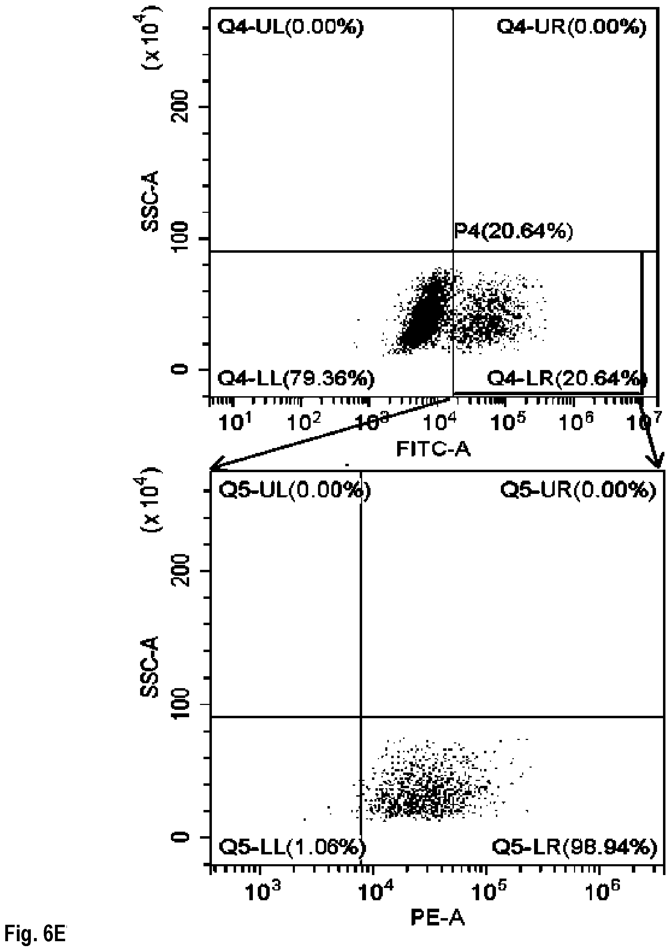

We hope to select bispecific binding molecules that can bind Kv1.3^{high} T_{EM} cells with binding preference, rather than all β7^{high} T cells. Among these bispecific binding molecules constructed, ATF-L1, which has moderate affinity for β7, high affinity for K v 1.3, as well as strong inhibitory effects on cell adhesion and Kv1.3 potassium current, was selected for binding selectivity test. Considering the relatively low expression of Kv1.3 on human T cells under physiological conditions, lentiviral vector with highly expressed Kv 1.3-EGFP gene was constructed to better compare the selectivity of bispecific binding molecules between Kv1.3 and β7 (Figure 6A). Human PBMC cells were stimulated with PMA and ionomycin (as described in 4.1), and after more than three rounds of stimulation, the ratio of β7^{high} T cells were close to 100% (Figure 3). The β7^{high} T cells were then infected with lentivirus with highly expressed Kv1.3-EGFP gene sequences, and the cells were used to test the binding selectivity when the infection rate was greater than 20% (Figure 6B-E).

### 5.2 Selectivity experiment

Binding selectivity was performed on Kv1.3^{high} β7^{high} T cells (≥20%) and Kv1.3^{low} β7^{high} T cells by using flow cytometry. PE/R-Phycoerythrin (Abeam) was covalently conjugated to ATF-L1, and then T Cells were incubated with different concentration of PE-labeled ATF-L1 at 4°C for 1 hour, washed with DPBS 2-3 times to remove unbound antibody, and then the binding capacity for Kv1.3^{high} β7^{high} T cells and Kv1.3^{low} β7^{high} T cells was evaluated. Results showed that ATF-L1 at low and median concentration tended to bind Kv1.3^{high} β7^{high} T cells. The percentage of ATF-L1 bound to Kv1.3^{high} β7^{high} T cells was 2.4 ± 0.1, 1.7± 0.2, 1.3 ± 0.1 and 1.2 ± 0.1 times higher than that of antibody bound to Kv1.3^{low} β7^{high} T cells at 50 pM, 100 pM, 200 pM and 400 pM concentration, respectively (Fig. 7A left two columns, 7B). As the concentration decreased, the bispecific binding molecule was more inclined to bind to Kv1.3^{high} β7^{high} T cells. At 50 pM, the binding rate on Kv1.3^{high} β7^{high} T cells was more than twice of that on Kv1.3^{low} β7^{high} T cells. As the concentration increased, this binding preference gradually weakened. The reason may be that the proportion of T cells with high expression of β7 was close to 100%, while T cells with high expression of Kv1.3 was only 20%. Therefore, at high concentrations, a huge number of PE-labeled ATF-L1 bound to β7 on T cells, thus masking its preference binding to Kv1.3. To further test this notion, competition experiment was performed: T cells were incubated with different concentrations of PE-labeled ATF-L1 at 4°C for 30 minutes, and washed with DPBS. Then 10-fold concentration of unlabeled etrolizumab was added and incubated at 4°C for 1 hour. After washed 2-3 times with DPBS followed by resuspension, the positive rate of PE was detected by flow cytometry, and compared with groups without etrolizumab competition. Results showed that when comparing with Kv1.3^{high} β7^{high} T cells, PE-labeled ATF-L1 bound to Kv1.3^{low} β7^{high} T cells was more easily to be competed by etrolizumab as can be seen from the reduced positive rate of PE (see two columns on the right in Figure 7A). At 50 pM, the binding rate of PE-labeled ATF-L1 to Kv1.3^{high} β7^{high} T cells was 4 times higher than that of Kv1.3^{low} β7^{high} T cells (Figure 7B). These results show that the binding of ATF-1 to K v 1.3 is more selective than that of β7, making it more easily bind to T cells with high expression of Kv1.3.

### 6. In vivo activity of bispecific binding molecules in animal models

Human T cells infected with lentivirus, in which the rate of β7 expression was close to 100% and K v 1.3 expression was 10-20%, were adoptively transferred into the Balb/c nude mice with DSS-induced colitis or normal control mice via tail vein injection. The inhibitory effects of ATF-L1 on the migration of transferred human T cells to the colon tissues of the mice were then studied.

### 6.1 Establishment of DSS-induce colitis mouse model

7-8 weeks old Balb/c nude mice were used for modeling. Mice were divided into two groups based on body weight, namely the normal control group and the DSS model group. The mice in the normal control group were given normal drinking water every day, while the mice in the DSS model group were given drinking water containing 2% DSS every day for 5-7 days. Body weight changes and disease activity index (DAI) scores were recorded daily.

### 6.2 Adoptive transfer of human T cells

1 mg of ATF-L1, etrolizumab or PBS were administered through the tail vein on the day before the adoptive transfer. On the transfer day, human T cells were labeled with Celltrace Yellow (Invitrogen), and then incubated in complete medium containing 200 pM ATF-L1, etrolizumab or PBS respectively at 37°C and 5% CO₂ for 1 hours. Before the adoptive transfer, 200 ul of peripheral blood was collected from the orbits of mice in each group as blank control. Hochst 33324 (Abeam) was injected into the tail vein to label the cell nuclei. After 30 minutes, 2x10⁶ human T cells labeled with Celltrace Yellow were adoptively transferred into mice via the tail vein. 30 minutes later, blood was collected through the orbit, and Texas Red dextran 70000 (Invitrogen) were injected through the tail vein, then the mouse was ready for in vivo imaging of colonic tissue with two-photon microscope.

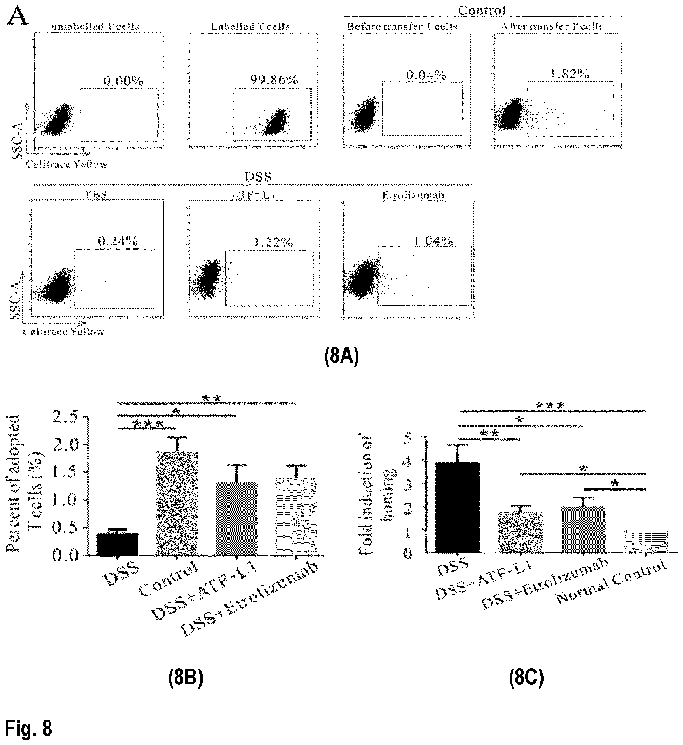

Peripheral blood collected before and after the adoptive transfer of cells was lysed with RBC lysis buffer from eBioscience TM, and then subjected for flow cytometry analysis. The results showed that the proportion of human T cells in PBMCs from the DSS model group was 0.439 ± 0.107%, which was significantly lower than that of other groups, indicating that the transferred cells may migrate to the site of intestinal inflammation; the proportion of human T cells in PBMCs from the blank control group was 1.987 ± 0.326% (p<0.001); in DSS group with ATF-L1 treatment, the proportion of human T cells was 1.3 ± 0. 0.3% (p<0.05); while the proportion of human T cells in DSS group with Etrolizumab treatment was 1.4 ± 0.2% (p<0.01) (Figure 8A-C).

### 6.3 Imaging of infiltrated human T Cells with high Kv1.3 expression in colon tissues

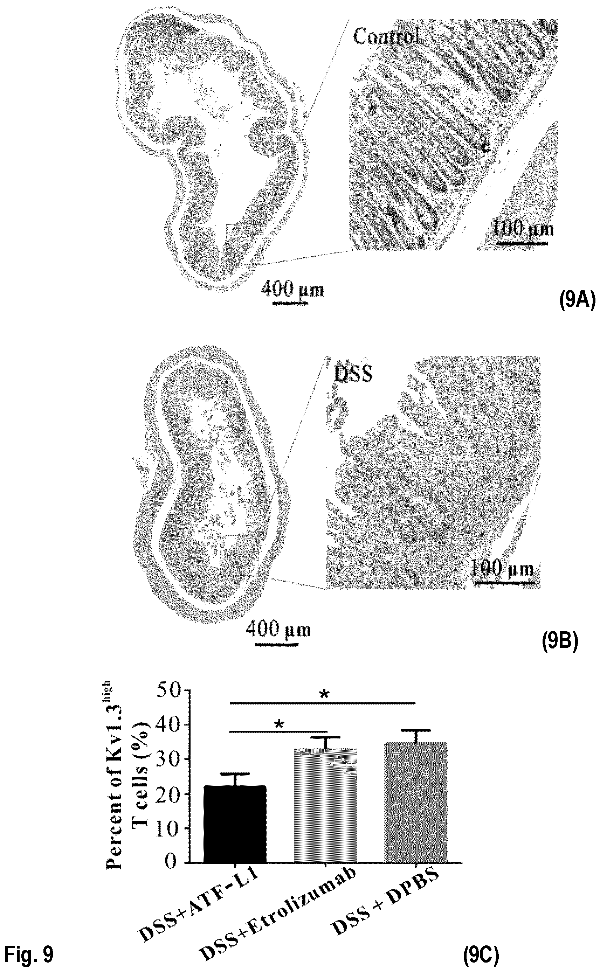

According to method reported in the literature (J Vis Exp.2012; (60):3678), the colon tissues of animals in each group were taken out, washed with saline, cut into 0.5-1cm segments, and then fixed with 4% paraformaldehyde for 24 hours. 100 µm sections of the fixed colon tissues from each group were prepared with a vibrating microtome (Leica VT1000S), and then were imaged with a Fv3000 (Olympus) fluorescent confocal microscope for analysis of the number of infiltrated Kv1.3^{high} T cells in each group. At the same time, 4 µm sections in paraffin were made for HE staining. Figure 9A-B showed that structure of the tissue sections from the DSS model animals displayed significant changes in comparison to that from the blank control group. The changes included destruction of the villi structure, a large number of immune cell infiltration, a large number of loss of goblet cells, and the absence of crypts. structure etc. The confocal imaging showed that a large number of infiltrated human T cells appeared in the lamina propria of the colonic mucosa in the DSS group in comparison to DSS plus drugs treatment group. The number of Kv1.3^{high} T cells in the DSS plus ATF-L1 treatment group was significantly lower than that in DSS plus etrolizumab treatment group and DSS model groups (Fig. 9C, p<0.05). The results showed that the bispecific binding molecule can specifically inhibit the infiltration of human Kv1.3^{high} T cell into inflammatory tissues in vivo.

### 7. Safety and efficacy of bispecific binding molecules

7-9 weeks old Balb/c mice were randomly divided into seven groups for safety and efficacy studies. Various doses of ATF-L1 or etrolizumab were administered to assess their effects on weight changes, disease activity index, and colon tissue length in DSS-induced colitis model mice. The results indicated that ATF-L1 effectively alleviated the weight loss and hampered the disease progression in the DSS-induced colitis model mice.

7~9-week-old Balb/c mice were randomly divided into 7 groups basing on body weight: ATL-1 10 mg/kg (low dose) group, ATL-1 15 mg/kg (high dose) group, etrolizumab 9.1 mg/kg (low dose) group, etrolizumab 13.6 mg/kg (high dose) group, panitumumab 14 mg/kg group, DSS model group, and the blank control group (DPBS group). All mice in DSS model group or DSS plus drug-treatment groups were given drinking water containing 2% DSS for 5 days and then normal drinking water without DSS for another 4 days, while the DPBS group were only given normal drinking water without DSS. Body weight changes and disease activity index (DAI) scores were recorded every day, and the grading criteria were shown in Table 1. All mice were sacrificed on the 9th day, and the colon tissues were taken for further analysis.

**Table 1: Detailed grading criteria for DAI**

| Clinical activity score | | | |
|---|---|---|---|
| Score | Weight | Stool consistency | Blood in stool |
| 0 | 0 | normal | No blood |
| 1 | 1-5% | soft | |
| 2 | 5-10% | very soft | Visual pellet bledding |
| 3 | 10-20% | diarrhea | |
| 4 | >20% | | gross bleeding, blood around anus |

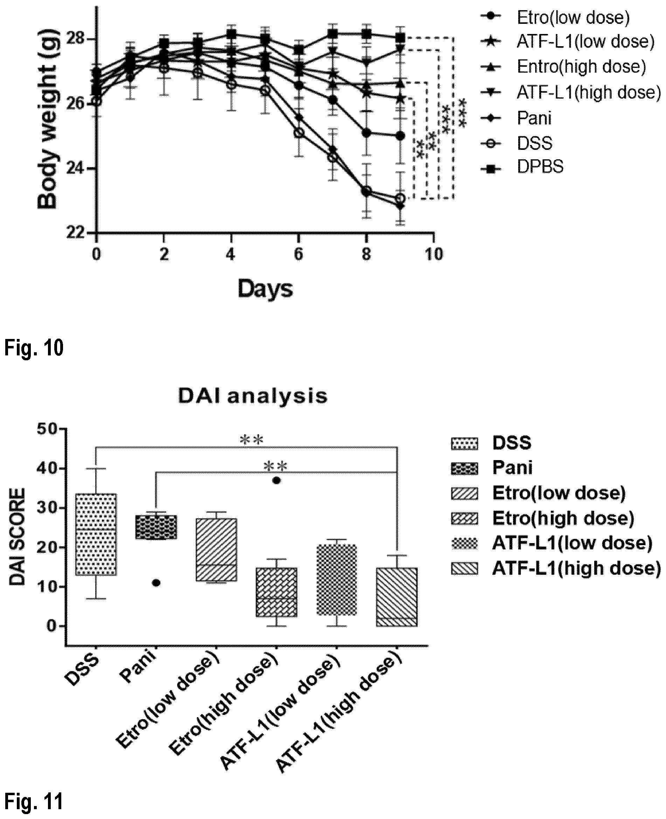

Results showed that the body weight changes in three groups (ATF-L1 10 mg/kg, ATF-L1 15 mg/kg and etrolizumab 13.6 mg/kg) were significantly different from that in DSS model group (Figure 10), indicating that both high and low dose of ATF-L1 and high dose of etrolizumab could effectively alleviate the weight loss of mice induced with DSS.

The DAI scores of each mouse in each group were summed up, and the differences among the groups were compared. As shown in Figure 11, significant differences were observed not only between ATF-L1 15 mg/kg group and DSS model group, but also between ATF-L1 15 mg/kg group and panitumumab group (14mg/kg) (**, P<0.01, one-way Anova test), suggesting that high dose of ATF-L1 significantly decreased the DSS-induced colonic inflammation in mice.

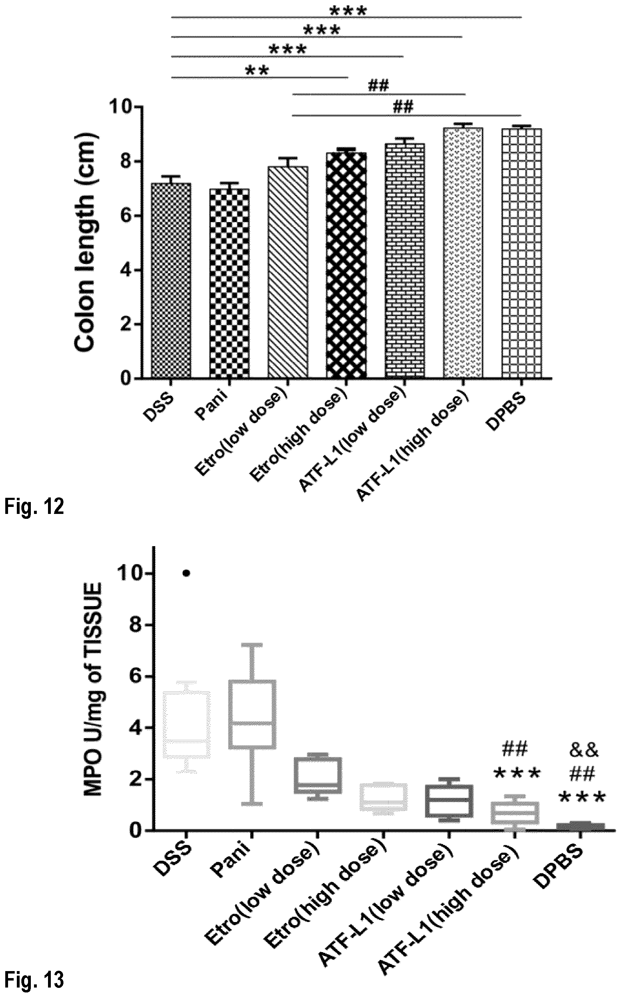

On the 9th day, the animals were euthanatized, the colon tissues was dissected, and the length of colons was measured. As shown in Figure 13, the colon length in all three groups (ATF-L1 10 mg/kg, ATF-L1 15 mg/kg and etrolizumab 13.6 mg/kg) were significantly different from that in DSS model group or in panitumumab group (*, P<0.05; ***, P<0.001; one-way Anova test). No significant difference was observed not only between ATF -L1 groups (10mg/kg and 15mg/kg) and DPBS group, but also between high-dose etrolizumab group (13.6mg/kg) and DPBS group. Results above implied that the 10 mg/kg and 15 mg/kg of ATF-L1 as well as 13.6 mg/kg of etrolizumab can significantly prevent the shortening of colon tissue in animals caused by DSS.

## Claims

1. A bispecific binding molecule comprising:
A) an inhibitory peptide targeting for K v 1.3 potassium ion channel;
B) an antibody specifically binding to integrin β7;
wherein the inhibitory peptide is fused to the antibody directly or through a linker peptide.

2. The bispecific binding molecule according to claim 1, wherein the amino acid sequence of A)peptide is grafted into within the amino acid sequence of B)antibody.

3. The bispecific binding molecule according to claim 2, wherein the amino acid sequence of A)peptide is grafted into the amino acid sequences of the heavy chain variable region (VH) and/or light chain variable region (VL) of B)antibody .

4. The bispecific binding molecule according to claim 3, wherein the amino acid sequence of A) peptide is grafted into the amino acid sequences of the heavy chain complementarity-determining region (HCDR) and/or light chain complementarity-determining region (LCDR) of B) antibody.

5. The bispecific binding molecule according to claim 4, wherein the amino acid sequence of A)peptide is grafted into the amino acid sequences of HCDR1 or HCDR3 of B)antibody .

6. The bispecific binding molecule according to claim 4, wherein the amino acid sequence of A)peptide is grafted into the amino acid sequences of LCDR1 or LCDR3 of B)antibody.

7. The bispecific binding molecule according to claim 3, wherein the amino acid sequence of A) peptide is grafted into the amino acid sequences of the framework region (FR) of B) antibody.

8. The bispecific binding molecule according to claim 7, wherein the amino acid sequence of A)peptide is grafted into the amino acid sequences of heavy chain FR3 and/or light chain FR3 of B)antibody.

9. The bispecific binding molecule according to any one of the preceding claims, wherein the molecule comprises heavy chain (HC) peptide and light chain (LC) peptide, wherein:
the HC peptide contains the constructs of A) peptide grafted into the heavy chain of B) antibody, and the LC peptide contains the light chain of B) antibody;
the LC peptide contains the constructs of A) peptide grafted into the light chain of B) antibody, and the HC peptide contains the heavy chain of B) antibody; or
the HC peptide contains the constructs of A) peptide grafted into the heavy chain of B) antibody, and the LC peptide contains the constructs of A) peptide grafted into the light chain of B) antibody.

10. The bispecific binding molecule according to any one of the preceding claims, wherein A)peptide is a toxin peptide derived from the Mexican scorpion (Vaejovis mexicanus).

11. The bispecific binding molecule according to any one of the preceding claims, wherein A)peptide is Vm24.

12. The bispecific binding molecule according to any one of the preceding claims, wherein A)peptide has an amino acid sequence as shown in SEQ ID NO: 9 (AAAISCVGSPECPPKCRAQGCKNGKCMNRKCKCYYC).

13. The bispecific binding molecule according to any one of the preceding claims, wherein A) antibody has the amino acid sequences of LCDR1 as shown in SEQ ID NO: 10, LCDR2 as shown in SEQ ID NO: 11, LCDR3 as shown in SEQ ID NO: 12, HCDR1 as shown in SEQ ID NO: 13, HCDR2 as shown in SEQ ID NO: 14, and HCDR3 as shown in SEQ ID NO: 15.

14. The bispecific binding molecule according to any one of the preceding claims, wherein A)antibody has the VH sequence from 1-117 amino acid residues as shown in SEQ ID NO: 1 and the VL sequence from 1-107 amino acid residues as shown in SEQ ID NO: 2.

15. The bispecific binding molecule according to any one of the preceding claims, wherein the linker peptide has a sequence as shown in GGSGAKLAALKAKLAALKGGGGS (SEQ ID NO: 16) or GGGGSELAALEAELAALEAGGSG (SEQ ID NO: 17).

16. The bispecific binding molecule according to claim 15, comprising:
HC sequences selected from SEQ ID NO: 1, 6, 7, 8, and
LC sequences selected from SEQ ID NO: 2, 3, 4, 5.

17. The bispecific binding molecule according to claim 16, comprising:
HC sequences as shown in SEQ ID NO: 1 and LC sequences as shown in SEQ ID NO: 3;
HC sequences as shown in SEQ ID NO: 1 and LC sequences as shown in SEQ ID NO: 5;
HC sequences as shown in SEQ ID NO: 6 and LC sequences as shown in SEQ ID NO: 2;
HC sequences as shown in SEQ ID NO: 8 and LC sequences as shown in SEQ ID NO: 2;
HC sequences as shown in SEQ ID NO: 1 and LC sequences as shown in SEQ ID NO: 4; or
HC sequences as shown in SEQ ID NO: 7 and LC sequences as shown in SEQ ID NO: 2.

18. A method for modifying an antibody specific to integrin β7 to enhance its therapeutic efficacy, comprising grafting a targeted inhibitory peptide for K v 1.3 potassium ion channel into the VH and/or VL regions of the antibody.

19. A method for providing a therapeutic agent of integrin β7 antibody, wherein the integrin β7 antibody comprising:
grafting a targeted inhibitory peptide for the Kv1.3 potassium ion channel into the VH and/or VL regions of the integrin β7 antibody to generate antibody variants;
screening for variant antibodies with higher selectivity for Kv1.3highβ7high T cells over Kv1.3lowβ7high T cells.

20. The method according to claim 18 or 19, wherein the antibody is the B) antibody according to any one of claims 1-17, and the targeted inhibitory peptide for the Kv1.3 potassium ion channel is the A) peptide according to any one of claims 1-17.

21. A nucleic acid encoding the bispecific binding molecule according to any one of claims 1-17.

22. An expression vector comprising the nucleic acid according to claim 10.

23. A host cell comprising the expression vector according to claim 22.

24. A method for producing the bispecific binding molecule according to any one of claims 1-17, comprising: culturing the host cell according to claim 23 under conditions suitable for the expression of the nucleic acid according to claim 21, and isolating the bispecific binding molecule.

25. A pharmaceutical composition comprising the bispecific binding molecule according to any one of claims 1-17 and a pharmaceutically acceptable carrier.

26. Use of the bispecific binding molecule according to any one of claims 1-17 in the preparation of a drug for treating or preventing conditions related to Kv1.3 potassium ion channel and/or integrin β7.

27. The use according to claim 26, wherein the conditions related to Kv1.3 potassium ion channel and/or integrin β7 include: inflammatory conditions, immune and proliferative diseases, rheumatoid arthritis (RA), ankylosing spondylitis, psoriatic arthritis, osteoarthritis, osteoporosis, uveitis, inflammatory fibrosis, scleroderma, pulmonary fibrosis, cirrhosis, inflammatory bowel disease, Crohn's disease, ulcerative colitis, asthma, allergic asthma, allergies, chronic obstructive pulmonary disease (COPD), multiple sclerosis, psoriasis, contact-mediated dermatitis, systemic lupus erythematosus (SLE) and other forms of lupus, diabetes, type I diabetes, obesity, cancer, lupus, restenosis, systemic sclerosis, scleroderma, glomerulonephritis, dry syndrome, inflammatory bone resorption, transplant rejection, or graft-versus-host disease.
